(19) Europäisches Patentamt / European Patent Office / Office européen des brevets

(11) **EP 4 445 924 A2**

(12) **EUROPEAN PATENT APPLICATION**

(43) Date of publication:
**16.10.2024 Bulletin 2024/42**

(51) International Patent Classification (IPC):
**A61M 1/14** *(2006.01)*

(21) Application number: **24195595.4**

(22) Date of filing: **28.08.2017**

(52) Cooperative Patent Classification (CPC):
**A61M 1/282; A61M 1/1524; A61M 1/155; A61M 1/1565; A61M 1/159; A61M 1/287**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2016 SE 1651180**

(62) Document number(s) of the earlier application(s) in accordance with Art. 76 EPC:
**17757771.5 / 3 506 963**

(71) Applicants:
• **Baxter International Inc.**
**Deerfield, IL 60015 (US)**

• **Vantive Health GmbH**
**8152 Glattpark (Opfikon) (CH)**

(72) Inventor: **Öberg, Carl**
**226 47 Lund (SE)**

(74) Representative: **Potter Clarkson**
**Chapel Quarter**
**Mount Street**
**Nottingham NG1 6HQ (GB)**

Remarks:
This application was filed on 21.08.2024 as a divisional application to the application mentioned under INID code 62.

(54) **APPARATUS FOR PROVIDING AUTOMATED PERITONEAL DIALYSIS**

(57)      An apparatus peritoneal dialysis (1) comprising an automated peritoneal dialysis cycler (3) programmed to run a treatment session including a plurality of cycles, each cycle including a fill phase, a dwell phase and a drain phase of a peritoneal cavity of a patient (P), the treatment session lasting at most 720 min; the cycler (3) has a patient line (54) in communication with the patient (P), a source (8) of treatment fluid which includes an osmotic agent, a pump (5) to circulate the treatment fluid and a control system (74) configured to drive the cycler (3) to deliver the treatment session. The control system (74) is programmed to run a fill phase of a first cycle delivering a first treatment fluid to the patient, the first treatment fluid having a first concentration of the osmotic agent; subsequently to the first cycle, to run a fill phase of a second cycle delivering a second treatment fluid to the patient, the second treatment fluid having a second concentration of the osmotic agent different from the concentration of the first treatment fluid; subsequently to the second cycle, to run a fill phase of a third cycle delivering a third treatment fluid to the patient, the third treatment fluid having a third concentration of the osmotic agent different from the concentration of the second treatment fluid. The second concentration of the osmotic agent is lower than the first concentration of the osmotic agent and the third concentration of osmotic agent is higher than the second concentration of osmotic agent.

FIG.1

## Description

## Technical Field

The present invention relates to the field of automated peritoneal dialysis (APD). More specifically, it relates to an apparatus for the treatment of renal insufficiency configured for optimizing a treatment session with APD with regard to osmotic water transport (UF), small/middle molecule clearance and glucose absorption.

In particular, the invention may be used for reducing the 'metabolic cost' in terms of glucose absorption and/or to shorten the total treatment time of the APD treatment.

## Background of the Invention

The kidneys fulfil many functions, including the removal of water, the excretion of catabolites (or waste from the metabolism, for example urea and creatinine), the regulation of the concentration of the electrolytes in the blood (e.g. sodium, potassium, magnesium, calcium, bicarbonate, phosphate, chloride) and the regulation of the acid/base equilibrium within the body, which is obtained in particular by the removal of weak acids (phosphates, monosodium acids) and by the production of ammonium salts.

In individuals who have lost the use of their kidneys, since these excretion and regulation mechanisms no longer work, the body accumulates water and waste from the metabolism and exhibits an excess of electrolytes, as well as, in general, acidosis, the pH of the blood plasma shifting downwards, below 7.35 (the blood pH normally varies within narrow limits of between 7.35 and 7.45).

In the treatment of patients suffering from acute or chronic renal insufficiency, dialysis therapy may be needed. The two general categories of dialysis therapy are hemodialysis and peritoneal dialysis.

In hemodialysis, the patient's blood is cleansed by passage through an artificial kidney in an extracorporeal membrane system.

The blood treatment involves extracorporeal circulation through an exchanger having a semipermeable membrane (dialyzer, or filtration unit) in which the patient's blood is circulated on one side of the membrane and a dialysis liquid, comprising the main electrolytes of the blood in concentrations close to those in the blood of a healthy subject, is circulated on the other side.

Furthermore, a pressure difference is created between the two compartments of the dialyzer which are delimited by the semipermeable membrane, so that a fraction of the plasma passes by ultrafiltration through the membrane into the compartment containing the dialysis liquid. Besides that there is also a diffusional transport of permeable solutes due to concentration gradients across the membrane.

In peritoneal dialysis, dialysis fluid is infused into the patient's peritoneal cavity. This cavity is lined by the peritoneal membrane which is highly vascularized. Substances are removed from the patient's blood by diffusion across the peritoneal membrane into the dialysis fluid. Excess fluid, i.e. water is also removed by osmosis induced by a hypertonic dialysis fluid (i.e. ultrafiltration).

When an aqueous solution is instilled into the peritoneal cavity, the solute composition equilibrates with that of plasma by passive diffusion along concentration gradients. The dialysis fluid contains various amounts of osmotic agent, usually glucose, creating an osmotic gradient that moves fluid and to some extent solutes to the peritoneal cavity, by convection or 'solvent drag'. Ultrafiltration continues until the dialysate becomes virtually isotonic, after which the rate that fluid is absorbed into the circulation exceeds that of the ultrafiltration induced by transcapillary hydrostatic pressure gradient alone. Net solute and water removal during peritoneal dialysis have been shown to be reduced by dialysate absorption. Through these two processes, diffusion and osmotic ultrafiltration, appropriate quantities of solute metabolites and fluid need to be removed to maintain the patient's body fluid volumes and composition within appropriate limits.

There are various types of peritoneal dialysis therapies, including continuous ambulatory peritoneal dialysis ("CAPD"), automated peritoneal dialysis ("APD"), including tidal flow APD, and continuous flow peritoneal dialysis ("CFPD").

CAPD is a manual dialysis treatment. The patient connects manually an implanted catheter to a drain, allowing spent dialysate fluid to drain from the peritoneal cavity. The patient then connects the catheter to a bag of fresh dialysis fluid, infusing fresh dialysis fluid through the catheter and into the patient. The patient disconnects the catheter from the fresh dialysis fluid bag and allows the dialysis fluid to dwell within the peritoneal cavity, wherein the transfer of waste, toxins and excess water takes place. After a dwell period, the patient repeats the manual dialysis procedure, for example, four times per day, each treatment lasting about an hour. Manual peritoneal dialysis requires a significant amount of time and effort from the patient, leaving ample room for improvement.

Automated peritoneal dialysis ("APD") is similar to CAPD in that the dialysis treatment/therapy includes drain, fill, and dwell cycles. APD machines, however, perform the cycles automatically, typically while the patient sleeps. APD machines free patients from having to manually perform the treatment cycles and from having to transport supplies during the day. APD machines connect fluidly to an implanted catheter, to a source or bag of fresh dialyzing fluid and to a fluid drain. APD machines pump fresh dialysis fluid from the dialysis fluid source, through the catheter, into the patient's peritoneal cavity and allow the dialysis fluid to dwell within the cavity and the transfer of waste, toxins and excess water to take place. APD machines pump spent dialysate from the peritoneal cavity, though the catheter, to the drain. As with the manual process, several drain, fill and dwell cycles occur during APD. A "last fill" often occurs at the end of the APD treatment, which remains in the peritoneal cavity of the patient until the next treatment.

Compared to conventional techniques, e.g. continuous ambulatory peritoneal dialysis (CAPD), APD offers the possibility to use increased dialysate flow rates (DFR) which would either be impractical or impossible to accomplish manually. Increasing the dialysate flow rate (DFR) by using more frequent exchanges typically improves the efficiency of APD. However, an increased DFR will increase the time spent filling and draining the peritoneal cavity, reducing the efficiency of the dialysis at higher DFRs. Thus, too frequent exchanges will reduce the efficiency of the dialysis and lead to a reduced cost/efficiency due to the increased consumption of dialysis fluid.

There are three exchange techniques of peritoneal dialysis, intermittent (IPD), tidal (TPD) and continuous (CFPD) technique. The latter requires the use of dual catheters and has only rarely been used. In IPD, each dwell is followed by a complete drain after which the peritoneal cavity is filled again with fresh dialysate. In TPD, after an initial fill volume (of usually e.g. 2L), only a portion of the initial fill volume is drained and replaced by fresh dialysis fluid during each cycle. Thus, there is always a certain amount of dialysate (the 'reservoir volume') that stays in contact with the peritoneal membrane throughout the dialysis session, after which the peritoneal cavity is drained completely. A prescription of TPD is usually defined by the percentage of the initial fill volume drained from the patient, e.g. 50% tidal APD for a 2L initial volume means that 1L is cycled with 1L reserve volume remaining in the peritoneal cavity.

Normal automated peritoneal dialysis (APD) is based on the use of ready to use fluids contained in bags of 5-6 L each that can be attached to an APD machine or cycler and administered to patients during one night of treatment. In general, at each filling step a certain amount of fresh fluid from a bag is filled into the patient peritoneal cavity and exchanges within it; thereafter a certain amount of spent fluid is drained from the peritoneal cavity and fresh fluid is supplied from the bag.

Peritoneal dialysis generally requires large volumes of dialysis fluid. Generally, at each application, or exchange, a given patient will infuse up to 2 to 3 liters of dialysis fluid into the peritoneal cavity. The fluid is allowed to dwell for approximately 1-3 hours, at which time it is drained out and exchanged for fresh fluid. Generally, four or more such exchanges are performed daily. Therefore, approximately 8 to 20 liters of dialysis fluid is required per day, 7 days a week, 365 days a year for each patient.

Dialysis fluids have traditionally been provided in sealed, heat sterilized form, ready for use. Peritoneal dialysis is typically performed using bags with three different concentration of glucose. The bags are being delivered to a patient's home as 1 L to 6 L bags with different glucose concentrations and a normal daily consumption is around 8-20 L of fluid.

Generally, the fluids used today have a glucose concentration in the range of e.g. 1.36%-4% w/v glucose; in other terms, bags are sold having different glucose content, the lower glucose content being around 1.36% in weigh of glucose in the bag, the higher glucose content is about 3.86% by weight of glucose in the bag. The used volumes during APD differs slightly depending on the markets, in US 12 L in total are the standard treatment, while in Europe it is more common to use 15 L of fluid.

Patients usually start peritoneal dialysis treatment with the lowest possible glucose strength available (e.g. 1.36% anhydrous glucose) for the single treatment session (e.g. 15 liters per treatment). Since peritoneum incurs physiological deterioration, at a certain point in time, glucose strength is increased, i.e. change one of the e.g. 1.36% bags to an e.g. 2.27% bag; timing for the change in glucose strength depends on the patient, but it occurs generally after several treatment sessions. Again after a certain number of treatments, glucose strength is increased again using two e.g. 2.27% bags and so on.

The increase in treatment efficacy is rather limited if the treatment volumes are increased above the 12-15 L used today.

It is further to be noted that shipping and storage of the sheer volume of fluids required is both inconvenient and expensive; further, the repeated connection and disconnection of many fluid containers creates a very substantial risk of biological contamination at the point of connection.

The response to these problems is to use small volumes of concentrated dialysis fluids diluted with purified water at the point of use (e.g. patient's home). Generally, such concentrates are separated into concentrated fluids containing the various appropriate electrolytes plus a buffer and concentrated fluids containing glucose or another appropriate osmotic agent. In other terms, two bags with concentrate of less than 1 L each replace approx. 15 L of peritoneal dialyzing (PD) fluid.

The various reasons for separating the dialysis fluid into two separate concentrates are known in the art and include concerns relating to sterilization and storage.

The compounding or proportioning of the concentrates with ultrapure water back into a finished, ready to use dialyzing fluid are generally performed by machine. Such machines require that the user attach the source of each concentrate to a particular inlet port on the proportioning apparatus, which may be integrated into the dialysis machine itself.

The preparation of ready to use PD fluids is done by dosing and mixing adequate volumes of concentrate and water in the heater bag.

The currently used peritoneal dialysis fluid for IPD and CAPD (being either pre-packaged or prepared from concentrates) which use glucose as the osmotic agent have some disadvantages. A first disadvantage is that high glucose or glucose concentration in the peritoneal dialysis fluid causes glucose to migrate through the peritoneum into the bloodstream, providing unwanted glucose calories and possibly elevated triglyceride levels to the patient (hypertriglyceridemia and/or obesity), and at the same time, reducing the osmolarity of the peritoneal dialysis fluid.

Moreover, the pH of the glucose-containing peritoneal dialysis fluid (typically in the pH range 5.2-5.5) is not as physiologic as some physicians may desire. Indeed, an abnormally low pH of the dialysis fluid may have possible irritating effects on the peritoneum.

EP 0402505 describes a continuous cycle peritoneal dialysis system including a source of sterilized dialysis fluid, and an inflow line and outflow line connected to a catheter implanted in the peritoneal cavity of a patient. A fill pump pumps sterilized dialysis fluid from the source to the catheter through the inflow line during a fill cycle. A drain pump pumps fluid from the catheter to a drain through the outflow line during a drain cycle. A volume of the dialysis fluid in the peritoneal cavity is detected and a process controller controls the fill and drain pumps in response to signals from a level detector to pump a calibrated fill volume of fluid into the cavity and a calibrated drain volume of fluid out of the cavity continuously in alternating fill and drain cycles in a manner that the fluid flows through the cavity generally with zero dwell time. In the process, an inflow volume and an outflow volume of dialysis fluid pumped into/out of the cavity is metered to determine the amount of fluid removed from the patient. A glucose pump is connected to a source of glucose and to the inflow line for delivering a regulated amount of glucose in response to a comparison of the metered inflow and outflow volumes. If the weight, i.e. fluid, is not being adequately removed, a drive signal is sent to glucose pump from controller to speed up the glucose pump, increase the glucose supplied to the dialysis fluid, and increase fluid removal. If too much weight is being removed, the glucose pump is slowed down to reduce the amount and ratio of glucose mixed with the fluid. The concentrate can easily be tailored to the patient by varying the glucose amount via pump and the glucose is automatically varied during the dialysis process to achieve a desired weight loss.

US 5643201 discloses a similar apparatus for peritoneal dialysis configured for monitoring the amount of water removed from the patient during dialysis and adjusting an amount of glucose mixed with the dialysis fluid to adjust the osmolality of the dialysis fluid and maintain a prescribed weight removal from the patient.

WO 2012/129501 describes a peritoneal dialysis system wherein a dialysis treatment includes multiple fill cycles, and the fill volumes and desired final concentrations of the osmotic agent may vary from cycle to cycle.

US 2008/125693 discloses a peritoneal dialysis system comprising an automated peritoneal dialysis (APD) machine programmed to run a therapy. In implementations including multiple cycles of treatment (e.g., multiple drain, fill, and dwell sequences), the concentration of glucose in custom dialysate can be altered from cycle to cycle. The glucose concentration of custom dialysate can, for example, be profiled throughout the treatment. In particular, the concentration of glucose in custom dialysate is gradually decreased from cycle to cycle. For example, custom dialysate can be produced to have a glucose concentration of about 4.25 percent (glucose monohydrate) during the first cycle, a glucose concentration of about 4.0 percent during a second cycle, a glucose concentration of about 3.0 percent during a third cycle, a glucose concentration of about 2.0 percent during a fourth cycle, and a glucose concentration of about 1.5 percent during a fifth cycle. As a result, the amount of water withdrawn from the patient during each cycle is gradually reduced from cycle to cycle. This technique helps to relieve the patient of discomfort associated with having built up and retained excessive amounts of water since the last treatment, and can prevent negative side effects that results from the removal of excessive amounts of water in the later stages of the treatment.

US 2014/221910 relates to a method for prescribing a dialysis treatment comprising the steps of collecting patient specific data, determining one target and defining, based on said patient specific data, a series of values V and t which allow to achieve the target (V represents the volume of dialysate used and t the duration of treatment). The method also includes displaying said series of values on a map. The method defines a procedure to deal with several bags of dialysate of different glucose concentrations to determine the choice of the bags of dialysate in order to fulfill a suitable target on ultrafiltration and minimize the patient's exposure to glucose.

The complete protocol manages the glucose charge and it is split in two parts, namely the minimization of glucose exposure, and the optimization by variable glucose concentration.

US 2010/7010426 discloses a peritoneal dialysis system comprising an automated peritoneal dialysis (APD) machine programmed to run a therapy. As discussed therein, one of the therapy parameters is percent glucose of the solution, which affects the amount of UF removed and the amount of calories absorbed by the patient. More glucose results in more UF removed, which is generally desirable. More glucose, however, results in more caloric intake and weight gain by the patient, which is not desirable. The APD machine described in connection with US 2010/010426 has the ability to connect to multiple solution bags having different glucose percentages and pull solution from the different bags to form a mixed or blended solution, e.g., in a warmer bag or in the patient's peritoneum if inline heating is used, having a desired glucose percentage different from the rated percentages, e.g., 2.0%, 2.88%, 3.38% and 3.81% glucose.

Notwithstanding more precisely controlling the glucose strength in the peritoneal dialysis treatment fluid delivered to the patient avoids excessive glucose overload due to (too high) hyperosmolarity of the treatment fluid, there is still a need to achieve proper and good fluid volume removal and small solute removal from the patient, at the same time, reducing the above mentioned negative effects of glucose absorption.

**Summary**

An aim of the present invention is providing apparatus for solving one or more of the cited prior art drawbacks.

In detail it is an aim of the present embodiment to provide an apparatus capable of shortening the total treatment time while achieving the same or better small-solute transport and UF.

A further aim of the invention is to make available an apparatus configured for reduce glucose absorption in the treatment session while achieving the same or better small-solute transport and UF.

It is an auxiliary aim of the invention to provide an apparatus capable of proportioning different treatment fluid with proper glucose content adapted to the treatment session and/or to the patient.

A further auxiliary aim of the invention is to make available an apparatus which may automatically determine the optimized treatment making use of a proper optimization algorithm making use of an extended three pore model having an additional compartment, allowing simulation also of the drain and fill phases of the dwell.

At least one of the above-indicated aims is attained by an apparatus as in one or more of the appended claims, taken singly or in any combination.

According to a first independent aspect, an apparatus for peritoneal dialysis (1) is provided comprising:

- an automated peritoneal dialysis cycler (3) programmed to run a treatment session including a plurality of cycles, each cycle including a fill phase, a dwell phase and a drain phase of a peritoneal cavity of a patient (P), in particular , the cycler (3) having:

  • a patient line (54) configured to be placed in communication with the peritoneal cavity of the patient (P);

  • at least one source (S) of treatment fluid which includes an osmotic agent;

  • at least one pump (5) to circulate the treatment fluid to be delivered to the patient via said patient line (54);

- a control system (74) configured to drive said cycler (3) to deliver the treatment session, wherein, during the same treatment session, the control system (74) is programmed to:

  ✔ run a fill phase of a first cycle delivering a first treatment fluid to the patient, the first treatment fluid having a first concentration of the osmotic agent;
  ✔ subsequently to the first cycle, run a fill phase of a second cycle delivering a second treatment fluid to the patient, the second treatment fluid having a second concentration of the osmotic agent different from the concentration of the first treatment fluid;
  ✔ subsequently to the second cycle, run a fill phase of a third cycle delivering a third treatment fluid to the patient, the third treatment fluid having a third concentration of the osmotic agent different from the concentration of the second treatment fluid;

  wherein the second concentration of the osmotic agent is higher than the first concentration of the osmotic agent and the third concentration of osmotic agent is lower than the second concentration of osmotic agent, or

  wherein the second concentration of the osmotic agent is lower than the first concentration of the osmotic agent and the third concentration of osmotic agent is higher than the second concentration of osmotic agent.

In a 2nd aspect according to the previous aspect, the osmotic agent is, or include, glucose, L-carnitine, glycerol, amino acid(s) or combinations thereof, in particular the osmotic agent being glucose.

In a 3rd aspect according to any of the previous aspects, the osmotic agent is glucose.

In a 4th aspect according to any of the previous aspects, the osmotic agent includes icodextrin.

In a 5th aspect according to any of the previous aspects, the control system (74) is programmed to run the first cycle followed by one or more additional cycles before running the second cycle and/or wherein the control (74) unit is programmed to run the second cycle followed by one or more additional cycles before running the third cycle, in particular one or more cycles of the treatment session being executed before the first cycle.

In a 6th aspect according to any of the previous aspects 1 to 4, the control system (74) is programmed to run the first cycle immediately followed by the second cycle and/or wherein the control system (74) is programmed to run the second cycle immediately followed by the third cycle.

In a 7th aspect according to any of the previous aspects, during said same treatment session, the control system (74) delivers a plurality of cycles using a treatment fluid with high concentration of osmotic agent, alternating with a plurality of cycles using a treatment fluid with low or no concentration of osmotic agent, said high concentration of osmotic agent being higher than said low concentration of osmotic agent.

In a 8th aspect according to the previous aspect 7, the treatment fluid with high concentration of osmotic agent includes a concentration of osmotic agent equal or higher than 3.0% in weight, optionally equal or higher than 4.0% in weight, and in more detail equal or higher than 5.0% in weight, said osmotic agent being in particular glucose.

In a 9th aspect according to any of the previous aspects 6 to 8, the treatment fluid with low concentration of osmotic agent includes a concentration of osmotic agent equal or lower than 1.5% in weight, optionally equal or lower than 1.0% in weight, and in more detail equal or lower than 0,5% in weight, in particular the treatment fluid with low or no concentration of osmotic agent includes a fluid without osmotic agents.

In a 10th aspect according to any of the previous aspects 6 to 9, in said plurality of cycles with high concentration of osmotic agent alternating with said plurality of cycles with low or no concentration of osmotic agent, a high concentration of osmotic agent cycle is followed by one or more subsequent cycles before a low or no concentration of osmotic agent cycle.

In a 11th aspect according to any of the previous aspects 6 to 10, in said plurality of cycles with high concentration of osmotic agent alternating with said plurality of cycles with low or no concentration of osmotic agent, a low or no concentration of osmotic agent cycle is followed by one or more subsequent cycles before a high concentration of osmotic agent cycle.

In a 12th aspect according to any of the previous aspect 6 to 9, in said plurality of cycles with high concentration of osmotic agent alternating with said plurality of cycles with low or no concentration of osmotic agent, a high concentration of osmotic agent cycle is immediately followed by a low or no concentration of osmotic agent cycle.

In a 13th aspect according to any of the previous aspects 6 to 9 and 12, wherein in said plurality of cycles with high concentration of osmotic agent alternating with said plurality of cycles with low or no concentration of osmotic agent, a low or no concentration of osmotic agent cycle is immediately followed by a high concentration of osmotic agent cycle.

In a 14th aspect according to any of the previous aspects, the control system (74) is configured to leave the treatment fluid in the peritoneal cavity for a dwell time, the dwell time of a treatment fluid with high osmotic agent concentration being different from, and in particular shorter than, a dwell time of a treatment fluid with low or no osmotic agent concentration, said high concentration of osmotic agent being higher than said low concentration of osmotic agent.

In a 15th aspect according to any of the previous aspects, the cycler (3) includes at least two sources (S) of treatment fluid, each source (S) being configured for feeding a treatment fluid different from another treatment fluid from another source (S) at least by the concentration of the osmotic agent, in particular each source (S) being a respective treatment fluid bag.

In a 16th aspect according to any of the previous aspects, the cycler (3) includes a disposable unit (3b) including said patient line (54) configured to be connected to the patient (P) and a peritoneal dialysis unit (3a) including said pump (5) and a compartment configured to receive the disposable unit (3b), the pump (5) being arranged so that when the disposable unit (3b) is disposed within the compartment, the pump (5) cooperates with the disposable unit (3b) to deliver the treatment fluid to and drain the treatment fluid from the peritoneal cavity of the patient through the patient line (54) of the disposable unit (3b).

In a 17th aspect according to the previous aspect, the disposable unit (3b) includes at least one inlet port (7; 8; 9) connected to said at least a source (S) of treatment fluid and at least one container (41) configured for receiving the treatment fluid from the source (S), in particular the cycler (3) being configured to heat the treatment fluid in the container (41) for receiving the treatment fluid before delivering to the patient via the patient line (54).

In a 18th aspect according to any of the previous aspects 16 or 17, the disposable unit (3b) includes at least two inlet ports (7, 8, 9), said inlet ports being respectively connected to sources (S) of treatment fluid having different concentration of osmotic agent, in particular a different concentration of glucose.

In a 19th aspect according to the previous aspects 17 or 18, the cycler (3) includes a valve device (71) to selectively put into communication one of the sources (S) of treatment fluid with the container (41) for receiving the treatment fluid, the control system (74) receiving a target concentration of osmotic agent in the treatment fluid and driving the valve device (71) and the pump (5) to withdraw a prefixed amount of treatment fluid from one of the sources (S) and a prefixed amount of treatment fluid from at least another of the sources (S) to obtain a treatment fluid in the container (41) for receiving the treatment fluid with a concentration of osmotic agent substantially matching the target concentration.

In a 20th aspect according to any of the previous aspects, the apparatus comprises a sensor (15) for sensing a parameter of the treatment fluid to be delivered to the patient via the patient line (54), the control system (74) being further configured to receive a signal from said sensor (15) and for determining the concentration of osmotic agent in the treatment fluid to be delivered to the patient.

In a 21st aspect according to the previous aspect, the sensor (15) is a concentration sensor for the osmotic agent, in particular a glucose concentration sensor.

In a 22nd aspect according to the previous aspect 20, the sensor (15) is a conductivity sensor, the treatment fluid having a conductivity which is linked to the osmotic agent concentration in a known relation, the control system (74) receiving the signal from the conductivity sensor (15) and determining the osmotic agent concentration using said known relation.

In a 23rd aspect according to the previous aspect 16, the apparatus comprises at least one source of a first concentrate (50), wherein the disposable unit (3b) includes:

- a water port (43); and

- at least a first inlet port (59) in fluid connection with said source of a first concentrate (50);

- at least a container (41) configured for receiving a treatment fluid prepared by mixing at least water and a first concentrate.

In a 24th aspect according to the previous aspect, the apparatus includes

- a water purifier (10) for preparing purified water and feeding to said water port (43),

- a water line (42) for feeding purified water to the water port (43) of the disposable unit (3b), said water line (42) being fluidly connected to the water purifier (10) to receive purified water; and

- a drain line (44) connected to a drain port (19) of the disposable unit (3b) to drain fluids at least from the disposable unit (3b);

- a sensor (15) for detecting a property of a fluid flowing in the drain line (44); and

- the control system (74) being configured to:

    ✓ sending the treatment fluid to be checked contained in the disposable unit (3b) into the drain line (44);
    ✓ pushing the treatment fluid to be checked to the sensor (15);

  wherein the control system (74) is further configured to achieve the pushing step by sending purified water along the water line (42) and directing said purified water from the water line into the drain line (44), said purified water in the drain line pushing the treatment fluid to be checked so as to reach the sensor (15).

In a 25th aspect according to any of the previous aspects, the control system (74) includes a memory storing a prediction algorithm, the control system (74), based on said prediction algorithm, being configured to determine at least the first concentration of osmotic agent in the first treatment fluid, the second concentration of osmotic agent in the second treatment fluid and the third concentration of osmotic agent in the third treatment fluid.

In a 26th aspect according to the previous aspect, the control system (74), based on said prediction algorithm, is configured to determine at least a first dwell time for the first treatment fluid, a second dwell time for the second treatment fluid and a third dwell time for the third treatment fluid.

In a 27th aspect according to any of the previous aspects 25 or 26, the control system (74) receives as input a desired total UF volume to be removed at the end of the treatment session and a desired total small solute removal, e.g. urea, to be removed at the end of said treatment session and provides said first, second and third concentrations for the osmotic agent as an output.

In a 28th aspect according to any of the previous aspects 25 to 27, the control system (74) receives as input the total number N of cycles of the treatment session and provides as an output, based on said prediction algorithm, a corresponding concentration of osmotic agent and a corresponding dwell time for each of the N cycles to achieve a desired total UF volume and total small solute volume targets at the end of the treatment session.

In a 29th aspect according to the previous aspects 24 to 26, the prediction algorithm includes a modified three pore model based on the equation:

$$\frac{dV_D}{dt} = J_{v,C} + J_{v,S} + J_{v,L} - L + J_{fill} - J_{drain}$$

wherein

$J_{v,C}$ is the net flow of water (in mL/min) across the aquaporines (transcellular pores);

$J_{v,S}$ is the net flow of water (in mL/min) across the highly selective pathways or "small pores";

$J_{v,L}$ is the net flow of water (in mL/min) across the weakly selective pathways or "large pores";

L is the net lymphatic clearance (in mL/min) from the peritoneal cavity to the circulation;

$J_{drain}$ is the flow of volume (in mL/min) to the source of treatment fluid; and

$J_{fill}$ is the flow of volume (in mL/min) from the source of treatment fluid.

In a 30th aspect according to any of the previous aspects, the control system is further configured to check a property of the treatment fluid using the sensor, in particular checking conductivity of the fluid.

In a 31st aspect according to any of the previous aspect 23 or 24, the port to which the drain line is connected is different from the water port, the control system being configured to direct said purified water from the water line into the drain line by moving the purified water entering into the disposable unit via the water port towards the drain port and to the drain line.

In a 32nd aspect according to any of the previous aspects 24 or 31, the control system is further configured to prepare said treatment fluid to be checked by:

- withdraw some purified water from the water line and send said purified water to the container configured for receiving a treatment fluid;

- withdraw some concentrate from the source of said first concentrate and send said first concentrate to the container configured for receiving a treatment fluid;

- optionally repeatedly withdraw some first mixed fluid from the container configured for receiving a treatment fluid and sending back to the container configured for improving mixing of the first mixed fluid, said first mixed fluid comprising said first concentrate from the first concentrate sources and said purified water,

said treatment fluid to be checked being said first mixed fluid.

In a 33rd aspect according to the previous aspect, the disposable unit has at least a second inlet port, at least one source of a second concentrate being in fluid connection with said second inlet port, the control system being configured to:

- withdraw some purified water from the water line and send said purified water to the container configured for receiving a treatment fluid;

- withdraw some concentrate from the source of said second concentrate and send said concentrate to the container configured for receiving a treatment fluid;

- optionally repeatedly withdraw some second mixed fluid from the container configured for receiving a treatment fluid and sending back to the container configured for improving mixing of the second mixed fluid, said second mixed fluid comprising said concentrate from the first and second concentrate sources and said purified water,

said fluid to be checked being said second mixed fluid.

In a 34th aspect according to the previous aspect, the control system is further configured to

- withdraw some purified water from the water line and send said purified water to the container configured for receiving a treatment fluid containing said second mixed fluid to dilute said second mixed fluid;

- optionally repeatedly withdraw some diluted second mixed fluid from the container configured for receiving a treatment fluid and sending back to the container configured for improving mixing of the diluted second mixed fluid;

said fluid to be checked being said diluted second mixed fluid.

In a 35th aspect according to the previous aspect, the control system, based on the diluted second mixed fluid property, is configured to

- withdraw some purified water from the water line and send said purified water to the container configured for receiving a treatment fluid containing said second mixed fluid to additionally dilute said second mixed fluid; and

- optionally repeatedly withdraw some additionally diluted second mixed fluid from the container configured for receiving a treatment fluid and sending back to the container configured for improving mixing of additionally diluted second mixed fluid;

said fluid to be checked being said additionally diluted second mixed fluid.

In a 36th aspect according to any of the previous aspects, the patient line is a single lumen or a double lumen tubing.

In a 37th aspect according to any of the previous aspects, the control system 74 comprises a control unit placed inside the cycler and controlling the cycler.

In a 38th aspect according to the previous aspect, the control system further includes an external controller communicating with the control unit to provide instructions to the control unit and/or providing data to and receiving data from the control unit.

In a 39th independent aspect a method for administering a peritoneal dialysis treatment is provided, the method comprises:

- providing a treatment session including a plurality of cycles, each cycle including a fill phase, a dwell phase and a drain phase of a peritoneal cavity of a patient (P), in particular the treatment session lasting at most 720 min;
- running a fill phase of a first cycle delivering a first treatment fluid to the patient, the first treatment fluid having a first concentration of the osmotic agent;
- subsequently to the first cycle, running a fill phase of a second cycle delivering a second treatment fluid to the patient, the second treatment fluid having a second concentration of the osmotic agent different from the concentration of the first treatment fluid;
- subsequently to the second cycle, running a fill phase of a third cycle delivering a third treatment fluid to the patient, the third treatment fluid having a third concentration of the osmotic agent different from the concentration of the second treatment fluid;

wherein the second concentration of the osmotic agent is higher than the first concentration of the osmotic agent and the third concentration of osmotic agent is lower than the second concentration of osmotic agent, or
wherein the second concentration of the osmotic agent is lower than the first concentration of the osmotic agent and the third concentration of osmotic agent is higher than the second concentration of osmotic agent.

In a 40th aspect according to the previous aspect, osmotic agent is, or include, glucose, L-carnitine, glycerol, or combinations thereof, in particular the osmotic agent being glucose.

In a 41st aspect according to any of the previous aspects 39 or 40, the first cycle is followed by one or more additional cycles before the second cycle and/or the second cycle is followed by one or more additional cycles before the third cycle.

In a 42nd aspect according to any of the previous aspects 39 or 40, the first cycle is immediately followed by the second cycle and/or the second cycle is immediately followed by the third cycle.

In a 43rd aspect according to any of the previous aspects 39 to 41, during said same treatment session, a plurality of cycles using a treatment fluid with high concentration of osmotic agent are delivered, alternating with a plurality of cycles using a treatment fluid with low or no concentration of osmotic agent, said high concentration of osmotic agent being higher than said low concentration of osmotic agent.

In a 44th aspect according to the previous aspect, the treatment fluid with high concentration of osmotic agent includes a concentration of osmotic agent equal or higher than 2,5% in weight, optionally equal or higher than 3% in weight, and in more detail equal or higher than 4% in weight, and in even more detail equal or higher than 5% by weight, said osmotic agent being in particular glucose.

In a 45th aspect according to any of the previous aspects 43 or 44, the treatment fluid with low concentration of osmotic agent includes a concentration of osmotic agent equal or lower than 2% in weight, optionally equal or lower than 1.5% in weight, and in more detail equal or lower than 0.5% in weight, said osmotic agent being in particular glucose.

In a 46th aspect according to any of the previous aspects 43 to 45, in said plurality of cycles with high concentration of osmotic agent alternating with said plurality of cycles with low or no concentration of osmotic agent, a high concentration of osmotic agent cycle is followed by one or more subsequent cycles before a low or no concentration of osmotic agent cycle.

In a 47th aspect according to any of the previous aspects 43 to 46, in said plurality of cycles with high concentration of osmotic agent alternating with said plurality of cycles with low or no concentration of osmotic agent, a low or no concentration of osmotic agent cycle is followed by one or more subsequent cycles before a high concentration of osmotic agent cycle.

In a 48th aspect according to any of the previous aspects 43 to 45, in said plurality of cycles with high concentration of osmotic agent alternating with said plurality of cycles with low or no concentration of osmotic agent, a high concentration of osmotic agent cycle is immediately followed by a low or no concentration of osmotic agent cycle.

In a 49th aspect according to any of the previous aspects 43 to 45 and 48, in said plurality of cycles with high concentration of osmotic agent alternating with said plurality of cycles with low or no concentration of osmotic agent, a low or no concentration of osmotic agent cycle is immediately followed by a high concentration of osmotic agent cycle.

In a 50th aspect according to any of the previous aspects 39 to 49, the method includes leaving the treatment fluid in the peritoneal cavity for a dwell time, the dwell time of a treatment fluid with high osmotic agent concentration being different from, and in particular lower than, a dwell time of a treatment fluid with low or no osmotic agent concentration.

In a 51st aspect according to any of the previous aspects 39 to 50, the method includes determining the osmotic agent concentration in the treatment fluid.

In a 52rd aspect according to the previous aspect, a conductivity sensor is provided for measuring conductivity of the treatment fluid, the method comprising determining the osmotic agent concentration in the treatment fluid based on the signal from the conductivity sensor.

In a 53rd aspect according to any of the previous aspects 39 to 52, the method includes determining at least the first concentration of osmotic agent in the first treatment fluid, the second concentration of osmotic agent in the second treatment fluid and the third concentration of osmotic agent in the third treatment fluid by means of a prediction algorithm.

In a 54th aspect according to the previous aspect, the method includes determining at least a first dwell time for the first treatment fluid, a second dwell time for the second treatment fluid and a third dwell time for the third treatment fluid by means of the prediction algorithm.

In a 55th aspect according to any of the previous aspects 53 or 54, the method includes receiving a desired total UF volume to be removed at the end of the treatment session and/or a desired total small solute removal, e.g. urea, to be removed at the end of said treatment session and providing said first, second and third concentrations for the osmotic agent.

Further characteristics and advantages of the present invention will better emerge from the detailed description that follows of at least some embodiments of the invention, illustrated by way of non-limiting example in the accompanying figures of the drawings.

## Brief Description of the Drawings

The description will now follow, with reference to the appended figures, provided by way of non-limiting example, in which:

Figure 1 schematically represents an apparatus for peritoneal dialysis made according to an illustrating embodiment;

Figure 1a shows a disposable unit including a cassette to be used with the apparatus of figure 1 or 2;

Figure 1b schematically shows a hydraulic circuit in the disposable cassette of the automated peritoneal dialysis cycler;

Figure 2 schematically represents an apparatus for peritoneal dialysis made according to an alternative illustrating embodiment;

Figure 3 schematically represents an apparatus for peritoneal dialysis made according to another illustrating embodiment;

Figures 4 is a perspective view of a peritoneal blood vessel showing the different sizes of pores used in a three pore model for predicting peritoneal dialysis therapy results;

Figure 5 shows simulated urea clearances as a function of DFR for the different techniques (IPD, TPD75/50/25), different transport types: Fast (lines labeled A), Average (lines labeled C), Slow (lines labeled B) and three different glucose concentrations: 1.5% (dotted line), 2.27% (solid line) and 3.86% (dashed line);

Figure 6 shows osmotic water transport (UF) per session hour as a function of DFR for the different techniques (IPD, TPD75/50/25), different transport types: Fast (lines labeled A), Average (lines labeled C), Slow (lines labeled B) and three different glucose concentrations: 1.5% (dotted line), 2.27% (solid line) and 3.86% (dashed line);

Figure 7 shows osmotic water transport (UF) in mL per gram glucose absorbed (or "UF efficiency") plotted as a function of DFR for the different techniques (IPD, TPD75/50/25), different transport types: Fast (lines labeled A), Average (lines labeled C), Slow (lines labeled B) and three different glucose concentrations: 1.5% (dotted line), 2.27% (solid line) and 3.86% (dashed line);

Figure 8 shows the small solute transport efficiency (in mmol urea removed per g glucose absorbed) as a function of DFR for the different techniques (IPD, TPD75/50/25), different transport types: Fast (lines labeled A), Average (lines labeled C), Slow (lines labeled B) and three different glucose concentrations: 1.5% (dotted line), 2.27% (solid line) and 3.86% (dashed line);

Figure 9 shows the clearance of $\beta_2$-microglobulin as a function of DFR for the different techniques (IPD, TPD75/50/25), different transport types: Fast (lines labeled A), Average (lines labeled C), Slow (lines labeled B) and three different glucose concentrations: 1.5% (dotted line), 2.27% (solid line) and 3.86% (dashed line);

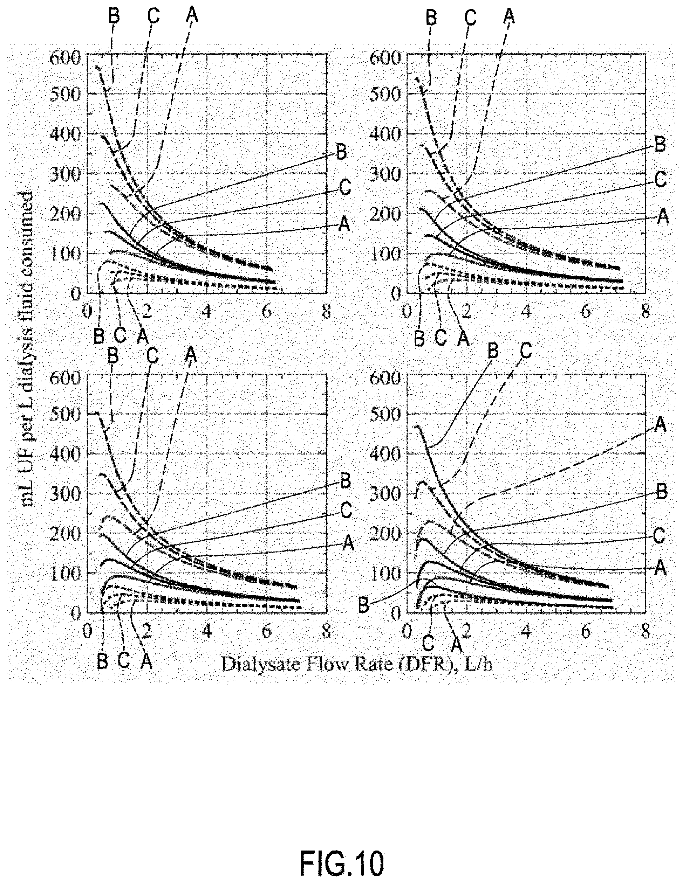

Figure 10 shows osmotic water transport (UF) in mL per liter dialysis fluid "consumed" as a function of DFR for the different techniques, transport types and different glucose concentrations: Fast (lines labeled A), Average (lines labeled C), Slow (lines labeled B) and three different glucose concentrations: 1.5% (dotted line), 2.27% (solid line) and 3.86% (dashed line);

Figure 11 shows simulated scenarios where each dwell is optimized for either UF (using 3.86% glucose) or small-solute transport (using 0% glucose) keeping the glucose absorption low (the corresponding transport parameters are shown in Table 3);

Figure 12 shows other simulated scenarios where each dwell is optimized for either UF (using 6% or 3.86% glucose) or small-solute transport (using 0% or 0,55% glucose) keeping the glucose absorption low.

## Detailed Description

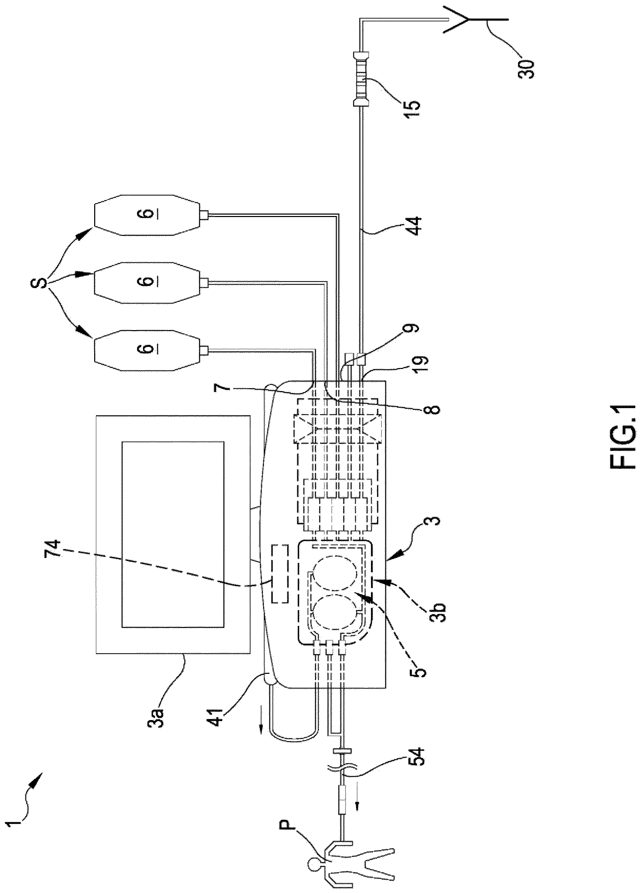

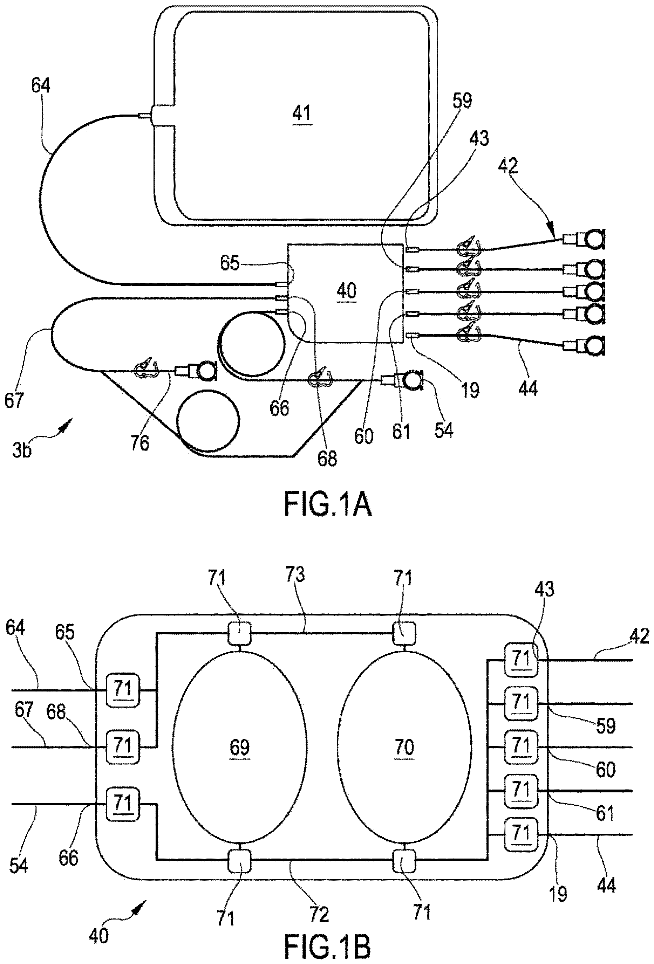

Figure 1 illustrates an apparatus 1 for peritoneal dialysis treatment in an embodiment of the invention.

The apparatus 1 is generally intended for the on-site treatment of a patient P with proper treatment fluid/s.

More in detail the system is designed for treating patients suffering of renal insufficiency, particularly with an automated peritoneal dialysis (APD) cycler 3.

The APD cycler 3 comprises a peritoneal dialysis (PD) unit 3a and a corresponding disposable unit 3b. The PD unit 3a includes a control system 74 and all the sensors and actuators to properly move the fluids inside the hydraulic circuit of the disposable unit 3a, as well as a user interface to e.g. input/output of data.

The disposable unit 3b (represented in figure 1a) is connectable on the PD unit 3a and includes a patient line 54 to be connected to a catheter implanted in the patient P to allow fill and drain operations of the peritoneal cavity of the patient him/herself.

The apparatus for peritoneal dialysis 1 includes two or more sources S of treatment fluid connected by means of respective feeding lines to corresponding treatment ports 7, 8, 9 of the disposable unit 3a.

Figure 1 shows three sources S of treatment fluid in the form of collapsible bags 6 filled with respective dialysate solutions. The content of each of the bag 6 is intended to be delivered to the peritoneal cavity of the patient, eventually after being heated.

In particular at least two (and eventually each) of said sources S include treatment fluids differing one from the other at least in respect to the concentration of an osmotic agent contained therein.

It is noted that the osmotic concentration of the treatment fluid relative to the blood determines to what extent fluids are exchanged between the PD solution and the blood. A high osmotic concentration in the treatment fluid creates a high gradient.

In any of the described embodiments, the osmotic agent may be, or include, glucose (or polyglucose), L-carnitine, glycerol, icodextrin, or any other suitable agents. Alternative osmotic agents may be fructose, sorbitol, mannitol and xylitol.

Glucose at concentration of 1.36%, 2.27% and 3.86% weigh/volume are commonly used. Alternatively glucose at concentrations of 0.5%, 1.5%, 2.5%, and 4.25% (296, 347, 397, and 485 mOsm/L respectively) are used. Glucose is continuously absorbed during dialysis which reduces the osmotic gradient and leads to a decline in ultrafiltration over time of the dwell.

Another osmotic agent used is polyglucose 7.5% (isoosmolar). Polyglucose is absorbed at a slower rate than glucose, which makes it suitable for long dwells.

As to peritoneal dialysis treatment solutions for the bag 6, Dianeal® solution, Nutrineal® solution, Extraneal® solution or Physioneal® solution, currently marketed by Baxter may be used.

Dianeal® solution has different glucose content, namely 0.5%, 1.5%, 2.5% and 4.5% (weight/volume) glucose.

Nutrineal® solution is glucose free, while Extraneal® solution uses icodextrin instead of glucose as a primary osmotic agent.

Physioneal® solution has different glucose content, namely 1.36%, 2.27% and 3.86% (weight/volume) glucose.

Though not limiting, in general treatment fluids for peritoneal dialysis have a composition including:

Na between 130 and 140 mmol/L;

Mg between 0.25 and 0.5 mmol/L;

Ca between 1.25 and 1.75 mmol/L;

Lactate between 30 and 40 mmol/L; alternatively lactate and bicarbonate with a total buffer concentration between 30 and 40 mmol/L (e.g. 15 lactate and 25 bicarbonate);

Glucose between 1.36% and 4% weight/volume as osmotic agent; or Glucose between 0.5% and 4% weight/volume as osmotic agent.

It is noted that glucose is also sometimes named as dextrose in the PD field. The term glucose is herewith intended to comprise dextrose, too.

As mentioned, some solution may be osmotic agent free or include icodextrin instead of glucose as osmotic agent, particularly for treatment solution destined for long dwell.

Moving back to figure 1, the bags 6 contains treatment fluids with different osmotic agent concentration, e.g. one bag with no glucose and the other with 2.27% and 3.86% glucose (anhydrous). Of course more than three bags 6 may be connected to different inlet ports of the disposable unit 3b. Also more than one bag with identical composition may be connected to the same inlet port, for example connecting the respective tubes with an "Y" connector.

In a first working example, the content of one of the connected bags 6 is delivered via a pump 5 to a container 41 for receiving a treatment fluid where the treatment fluid is properly heated and thereafter delivered to the patient P via patient line 54 for a fill, dwell and drain cycle. The subsequent cycle, depending on the treatment session prescription (as will be detailed in the following part of the description), may use the same treatment fluid, or a treatment fluid from a different bag 6 (i.e. with different osmotic agent concentration). The plurality of cycles of a treatment session are automatically executed by the control system delivering the prescribed treatment fluid with specific osmotic agent content for each cycle.

In a different working example, it is possible to properly mix the content of different bags 6 to generate a treatment fluid with a concentration of osmotic agent different from the concentration of any of the prepackaged bags 6 so that the concentration of osmotic agent (e.g. glucose) may be varied before treating the patient. As discussed, one of the therapy parameters is percent osmotic agent (e.g. glucose) of the solution, which affects the amount of UF removed and the amount of calories absorbed by the patient. More glucose results in more UF removed, which is generally desirable. More glucose, however, results in more caloric intake and weight gain by the patient, which is not desirable.

Glucose profiling is therefore an important factor in selecting the patient's possible therapies. For example, if using Dianeal® fluid, the different solutions are provided in different glucose percentages, such as 1.36%, 2.27% and 3.86% glucose. The APD machine described in connection with the present apparatus has the ability to connect to multiple solution bags having different glucose percentages and pull solution from the different bags to form a mixed or blended solution, e.g., in the container 41 or in the patient's peritoneum if inline heating is used, having a desired glucose percentage different from the rated percentages, e.g., 2.0%, 2.88%, 3.38% and 3.81% glucose.

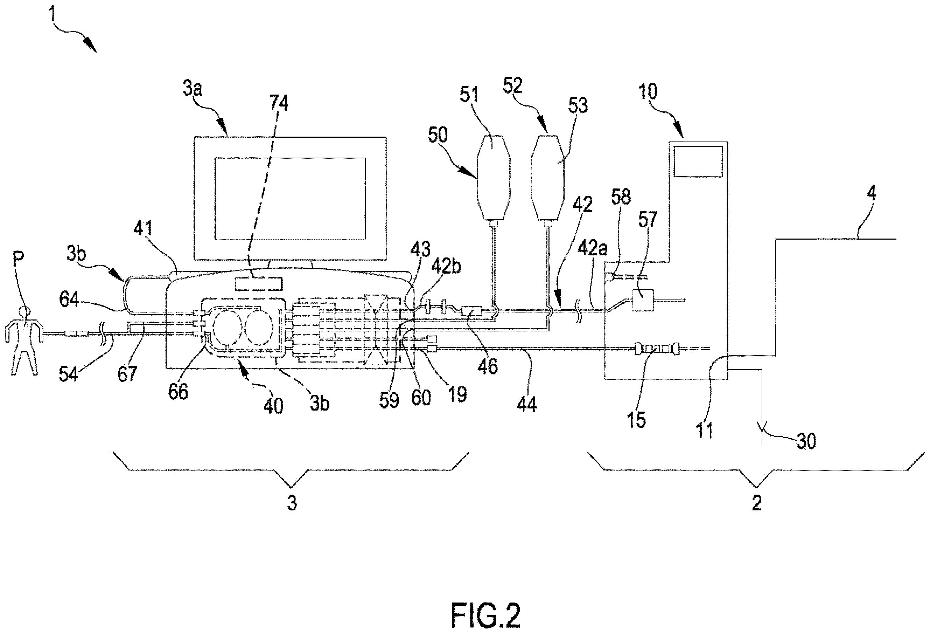

Alternatively, sensor 15 may be positioned on a drain line 44 to sense proper mixing of the mixed solution with a specific testing procedure described in respect to the apparatus of figure 2.

Figure 2 illustrates an alternative embodiment of the apparatus of figure 1.

The apparatus 1 depicted in figure 2 is generally intended for the on-site preparation of treatment fluids and for the treatment of the patient with the prepared fluids.

In other terms, the apparatus 1 is generally configured for preparing a peritoneal dialysis treatment fluid by mixing purified water (e.g. on site prepared) and concentrates and for treating a patient in a peritoneal dialysis treatment.

Therefore, the apparatus according to figure 2 mainly differs from the previously described APD cycler because no use is made of different bags (or sources S) containing respective treatment fluids ready to be infused into the patient peritoneal cavity.

In the apparatus of figure 2, the treatment fluid has to be prepared properly mixing concentrates and water; therefore, the source S of treatment fluid is container 41 where the treatment fluid is obtained.

The apparatus 1 includes a water preparation module 2 and the APD cycler 3. As per the case depicted in figure 1, the cycler 3 is made of a peritoneal dialysis (PD) unit 3a and of a disposable unit 3b particularly in the form of a cassette 40 to which a plurality of lines and a container 41 configured for receiving a treatment fluid are connected.

According to figure 2, the water preparation module 2 receives water from a water source 4, particularly a continuous water source such as the water pipelines of the water main.

In general, the water preparation module 2 is installed in a room having access to the water source 4 to provide purified water to the APD cycler 3 as apparent from below description.

Water enters a water purifier 10 via a water intake 11. The water purifier 10 may be a reverse osmosis unit (WRO unit) of known type not herein detailed.

The purified water leaves the WRO unit through the purified water outlet and goes to the APD cycler via a (purified) water line 42. A pressure regulator 57 may be positioned at the purified water outlet upstream the water line 42 along the water flow for regulating fluid pressure in the water line.

The water line 42 feeds purified water to a water port 43 of the cassette 40 of the disposable unit 3b. The water line 42 is a flexible tube having one first end 42a connected to an exit of the purifying circuit 12 of the water purifier 10 and a second end 42b connected to the water port 43 of the cassette 40.

The apparatus 1 also include a drain line 44 to bring fluid to a drain, for example the drain 30 of the water purifier 10.

Also the drain line 44 is a tube having one first end connected to the proportioning device 3 and a second end preferably connected to the purifying circuit 12 of the water purifier 10.

The water line 42 and the drain line 44 may (or may not) run parallel in the form of a dual lumen tubing, for example. In this respect, also the drain line 44 includes a flexible tube and is connected to a drain port 19 of the cassette 40.

In an advantageous embodiment, the water line 42 includes a first tract 42a and a second tract 42b connected to the first tract with a connector 46; the second tract 42b is connected to said water port 43 and may present a first and a second sterile filter. The two filters may be disposable filters as well. In particular the second tract 42b may be fixedly (but also removable when needed) connected to the cassette 40 and be a disposable part.

Notably, the water line 42 and the drain line 44 comprise a terminal connector (e.g. connector 46) configured for connecting a free end of the respective line to an intake 58 of the purifying circuit of the water purifier 10 for disinfection of the water and drain lines.

As exemplified in figure 1B showing a portion of the hydraulic circuit of the disposable unit 3b according to both figure 1 and figure 2 examples, respective ends of the water and of the drain line 42, 44 enters into the water port 43 and drain port 19, respectively; a fluid line exits the port 43 to direct fluid into the cassette 40 (or the disposable unit 3b) and a fluid line enters the drain port 19 to direct fluid out of the cassette 40 (or the disposable unit 3b).

The apparatus 1 further includes at least a sensor 15 for detecting a property of a fluid flowing in the disposable unit 3b (e.g. the patient line), water line 42 and/or in the drain line 44. The sensor 15 may be a sensor for detecting a property of the treatment fluid allowing the control system 74 to determine the osmotic agent concentration in the treatment fluid to be delivered to the patient P. As an example the sensor may be a concentration sensor for the osmotic agent, e.g. a glucose concentration sensor. Alternatively, the sensor 15 may be a conductivity sensor and may be placed in the drain line 44. Possibly, but not necessarily, the sensor 15 is included in the hydraulic circuit of the water purifier 10. Since osmotic agent (e.g. glucose) content is linked in a known manner with treatment fluid conductivity, the control system 74 receiving the signal form conductivity sensor 15 may determine the concentration of osmotic agent in the sensed treatment fluid.

Moving to the proportioning of the fluids (back to figure 2), the apparatus 1 further comprises at least one source 50 of a first concentrate in fluid connection with the first inlet port 59 of the proportioning device 3. In particular, the source 50 of the first concentrate is a first container 51; the container 51 may be used for several PD fluid preparation cycles and it is substituted only once all the concentrate contained therein has been completely used.

The first concentrate contains an appropriate osmotic agent, such as glucose. In a non-limiting example, the first concentrate includes 50% glucose at pH between 2 and 3. Preferred volumes may be comprised between 1 and 4 liters.

The apparatus 1 further comprises at least one source 52 of a second concentrate in fluid connection with the second inlet port 60 of the proportioning device 3. In particular, the source 52 of the second concentrate is a second container 53; the container 53 may be used for several PD fluid preparation cycles and it is substituted only once all the concentrate contained therein has been completely used.

The second concentrate contains electrolytes and a buffer agent, for example lactate. In a non-limiting example, the second concentrate includes sodium chloride, calcium chloride, magnesium chloride and sodium lactate at pH higher than 6. Preferred volumes may be comprised between 0,5 and 4 liters.

In general two concentrates containers 51, 53 will be used, however it is not excluded the possibility to use three or more concentrates.

For example, a source of a third concentrate in fluid connection with a third inlet port 61 of the disposable unit 3b may be provided. In particular, the source 62 of the third concentrate is a third container; the container may be used for several PD fluid preparation cycles and it is substituted only once all the concentrate contained therein has been completely used.

In case three concentrates are used, the second concentrate may, as an example, include sodium chloride, sodium lactate and sodium bicarbonate and the third concentrate may, as an example, contain other electrolytes, such as calcium and magnesium chloride.

Of course different content for the concentrates may be adopted depending on the patient need and on the circumstances.

As mentioned, the proportioning device 3 is made up of two main parts: the APD cycler 3a and the disposable unit 3b in the form of a disposable set which is generally replaced after each treatment session (see figure 1a). The disposable unit 3a of figures 1 and 2 is substantially identical and shown in figure 1a.

The disposable unit 3b includes a disposable cassette 40, as well as a set of tubes. As shown in figure 1a, the tubing set includes a container line 64 emerging from a container port 65 of the cassette and terminating in a container 41 configured for receiving a treatment fluid. The container 41 is in the form of a collapsible bag to be positioned e.g. in a dedicated tray on the cycler 3a (see figure 1).

The tubing set also includes a portion of the water line 42 and a portion of the drain line 44 both emerging from the respective water port 43 and drain port 19 and three (or more) line portions emerging from first, second and third concentrate ports 59, 60 and 61 configured for connection to respective concentrate bags. Figure 1a shows three line portions for connection to concentrates; figure 1 illustrates (on the right side) the water port 43, the drain port 19 and three additional ports in the cassette, two of them for connection to concentrates.

The patient line 54 emerges from a respective patient port 66; one end of the patient line 54 is configured for connection to a patient P. An additional line 67 emerging from an additional port 68 in the cassette 40 may be present, as currently shown. This additional line 67 may be used as an additional drain or (as shown) may have one end connected to the patient line 54 to create a loop.

It should be appreciated however that other lines associated with the cassette 40 may be provided.

The cassette 40 is also provided with a first and (possibly) a second pump chamber 69, 70 (see for example figure 1b). Each of the pump chambers is selectively in fluid communication with the described ports 43, 59, 60, 61, 19, 65, 66 and 68.

In particular the water port 43 (and therefore the water line 42 and the drain line 44), and the first, second and third port 59, 60, 61 (and therefore the concentrates) and the drain port 19 are fluidly connected to a first common line 72, this latter is fluidly connected to one side of the pump chambers 69, 70. Also the patient port 66 (shown e.g. on the opposite side of the cassette 40) is connected to the first common line 72.

The container port 65 (and therefore the container 41) and the additional port 68 are fluidly connected to a second common line 73, this latter is fluidly connected to the opposite side of the pump chambers 69, 70.

First common line 72 and second common line 73 communicate one with the other by means of the pump chambers 69, 70. In case the patient line 54 and the additional line 67 are connected to each other, there is a further communication path between common lines 72, 73.

In correspondence of each of the mentioned ports and also in correspondence of the respectively opposite fluid connections to the pump chambers (on line 72 and 73), at least one valve 71 is provided. See again figure 1b.

Acting on the valves 71 (e.g. opening and closing the passages) allows to selectively change fluid flow path inside the disposable unit 3b.

The cycler 3a receives the cassette 40 and the set of tubing. The cycler 3a is provided with a control system 74 driving respective valve actuators to opening or closing the each one of the valves and therefore 'creating' the different flow paths inside the hydraulic circuit.

The cycler 3a also has a respective actuator for each of the pump chamber 69, 70, active to push and release the membrane of the pump chamber thereby creating a fluid pressure inside the chamber and a corresponding fluid flow inside the fluid path of the disposable unit 3b.

Pump chambers and actuators define a first pump, in particular a membrane pump, for moving fluid towards and from the container 41 for receiving the treatment fluid (and moving fluids towards/from the requested ports) and a second pump, in particular a membrane pump, for moving fluid towards and from the container 41 for receiving the treatment fluid (and moving fluids towards/from the requested ports).

Once fluid connections have been made, the preparation of the treatment fluid for the patient treatment with the apparatus of figure 2 may start.

The water purifier 10 feeds purified water to the water line 42, the water port 43 is opened, purified water enters into the cassette 40 and wet the latter; water is directed into the container 41.

Then, the first concentrate (if not known) may be checked. Water port 43 is closed and first inlet port 59 is opened. The control system 74 acts on pumps and valves of the proportioning device to withdraw some concentrate from the source 50 of the first concentrate and send said concentrate into the cassette 40, particularly filling (at least partly) one of the pump chamber 69, 70.

First inlet port 69 is closed and drain port 19 is opened. The pump of the chamber containing the first concentrate is activated so that the first concentrate is forced through the drain port 19 towards and in the drain line 44. Some first concentrate reaches the first drain tract 49a.

In subsequent step, the control system 74 drives the APD cycler 3 to withdraw purified water form container 41; purified water from the container 41 is used to pushing forward the first concentrate in the drain line. First concentrate is forced through the drain line 44 towards the sensor 15.

In more detail, the control system 74 is configured to send the first concentrate into a first tract 49a of the drain line 44; said first tract is positioned (immediately) downstream the drain port 19. The control system pushes the first concentrate in the drain line 44 by means of purified water from the container 41 so that the first concentrate reaches the sensor 15 and simultaneously flush the pump chamber 70. The property of the first concentrate may then be measured and the first concentrate (i.e. the concentrate connected to the first inlet port 59) properly identified/checked.

A similar procedure is adopted for the second concentrate. Second concentrate is checked by sensor 54 and identified.

It is apparent that the identification steps are optional since the container identification may be left to the user and/or achieved through use of dedicated mechanical connectors which may not allow connecting a concentrate container different from the right container to the respective inlet port.

The apparatus is now ready for mixing the concentrates and water to produce PD treatment fluid with the desired osmotic agent concentration.

Firstly purified water is pumped to the container 41 from the water purifier thought the water port 43 and pump chambers 69, 70 via container line 64. First concentrate is then pumped into the container 41 via the first inlet port 59 and properly mixed.

Subsequently, additional purified water is supplied via the water line 42 to the container 41 so that pump chambers are rinsed with water and first mixed fluid still in the pump chamber moved to container 41. Second concentrate is added to container 41 via second inlet port 60, the pump chambers 69, 70 and the container line 64.

Once the required and metered quantity of the two concentrates has been supplied to the container 41, a main dilution phase starts. Purified water is added to the container configured form the RO water purifier 10, to reach about 90-95% of final fluid volume in the container 41.

The diluted second mixed fluid is then checked to verify that the fluids have been properly mixed and particularly that the osmotic agent concentration is the desired one. To check this, a sensor may be placed on the container 41 to check concentration of a specific substance (e.g. the osmotic agent) or to check conductivity of the prepared treatment fluid. Alternatively sensor 15 on the drain line 44 is used. One of the pumps 69 or 70, or both withdraws some diluted second mixed fluid from container and directs it into the first drain tract 49a via drain port 43.

In order not to waste treatment fluid, as soon as diluted second mixed fluid reaches the drain line 44, purified water is pushed by the water purifier 10 in the water line towards the drain line 44 thereby forcing the diluted second mixed fluid to the sensor 15 for fluid property check.

An auxiliary dilution step to fine tune the treatment fluid composition may be run if necessary. In particular, additional purified water is added to the container to further dilute. The amount of added purified water is calculated as a function of the measured property (e.g. conductivity) of the diluted second mixed fluid. In more detail the amount of added purified water is calculated as a function of the measured property also in relation to the previously filled amount of fluid (water and concentrates).

Conductivity of the additionally diluted second mixed fluid may then be checked to confirm correct preparation of the treatment fluid.

Some additionally diluted second mixed fluid is withdrawn from container 41 and fed to the drain line 44 and then purified water from water line 42 pushes the additionally diluted second mixed fluid to the sensor 15 for final check.

The apparatus is now ready for treating a patient according to the treating sessions/required cycles following a procedure according to the invention as explained in the final part of the present description. In general, the patient is connected, spent dialysate (if present) is removed from the peritoneal cavity and sent to drain 30 via drain line 44; fresh dialyzing fluid, on-site prepared and having the desired concentration of osmotic agent, is directed from container 41 (which is therefore the source S of treatment fluid) to the patient P. Once all the consecutive cycles (e.g. 1 or more cycles) with this specific treatment fluid are concluded, a second treatment fluid with a second concentration of osmotic agent is prepared according to the previously described preparation phases. The patient undergoes all the consecutive cycles required by the treatment prescription with the treatment fluid having the second concentration of osmotic agent. The treatment session proceeds with the preparation of the third treatment fluid having a third concentration of osmotic agent (different from the second concentration) and the respective cycle/cycles are administered to the patient.

It is noted that the disposable unit 3b may include additional bags (e.g. connected to the mentioned inlet ports of the cassette 40) which might be filled with treatment solution having a certain osmotic agent concentration previously prepared by the apparatus and to be used subsequently to another treatment fluid with different osmotic agent content.

In this respect, in case a treatment session requires two treatment fluids, only, with high and low (or no) osmotic agent (e.g. glucose) concentration to be alternated, once the first treatment fluid is prepared and used, the residual amount contained in the container 41 is directed and stored in a receiving additional bag connected to one port of the cassette 40. A second treatment fluid is prepared in the container 41, used and thereafter stored in a different additional bag connected to the cassette 40. Therefore, the first treatment fluid may be withdrawn from the receiving bag where it was stored, directed to the container 41 and used for further cycles while the residual second treatment fluid remains provisionally stored in another receiving bag for the subsequent use. Operating as above described allows to prepare each of the two fluids only once, but to administer treatment cycles to the patient P alternating the two treatment fluids.

At the end of the treatment session, the patient P is disconnected and the disposable unit 3b (e.g. the cassette 40 and connected tubing set) is disposed.

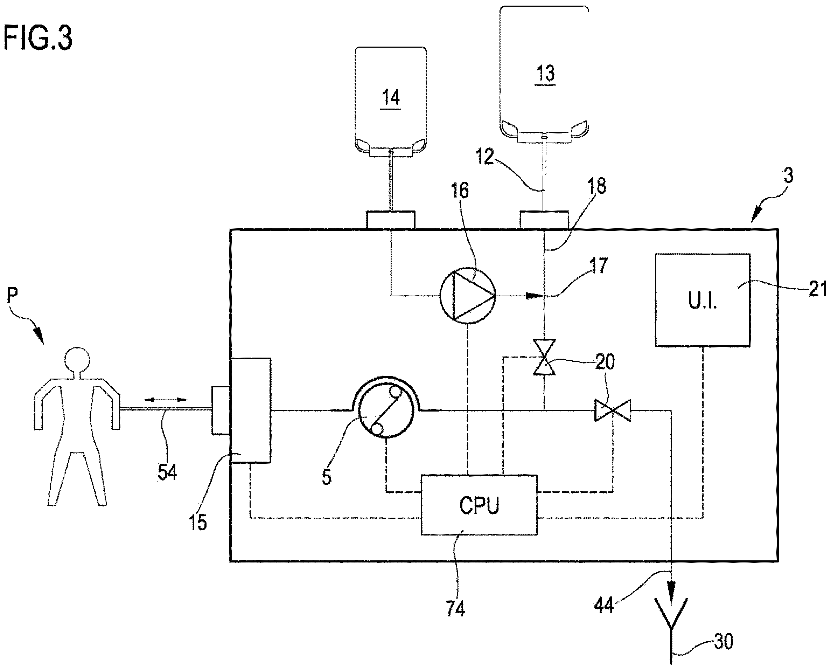

A third embodiment of a PD apparatus is very schematically shown in figure 3.

The PD cycler 3 receives a dialysis fluid from a feeding line 12; dialysis solution may be contained in a treatment solution container 13 or may be on-line prepared from water and concentrates as known in the art.

An osmotic agent concentrate container 14 is also provided connected to the cycler 3. A metering pump 16 feeds the osmotic agent (e.g. glucose) concentrate towards a mixing point 17 on a main line 18 circulating the dialysis solution. The dialysis solution and the osmotic agent concentrate mix properly to generate a PD treatment fluid with the desired concentration of osmotic agent. In this respect the source S of treatment fluid is the line downstream the mixing point 17.

A main pump 5 is used to direct the treatment fluid to the patient P via the patient line 54. A sensor 15 on the patient line measures a property of the treatment fluid allowing the control system 74 of the cycler to determine that the treatment fluid has the correct and desired concentration of osmotic agent. A feedback control loop on the metering 16 pump may be created to adjust osmotic agent concentration in case of deviations from the desired value.

The same main pump 5 may operate in reverse mode to drain fluid from the patient P directing PD treatment fluid along a drain line 44 towards a drain 30. In this respect, the control system 74 controls metering and main pump 5 and 16, as well as valves 20 to properly mixing and directing fluids. The control system 74 also receives signal from the sensor 15 and inputs/outputs from a user interface 21.

The apparatus of figure 3 allows to prepare any treatment fluid with a specifically desired concentration of osmotic agent.

## The extended three pore model

In order to determine an optimized APD treatment session with regard to maximizing UF and small solute transport and, at the same time, minimizing the metabolic cost in terms of glucose absorption, an extended three pore model has been developed and used.

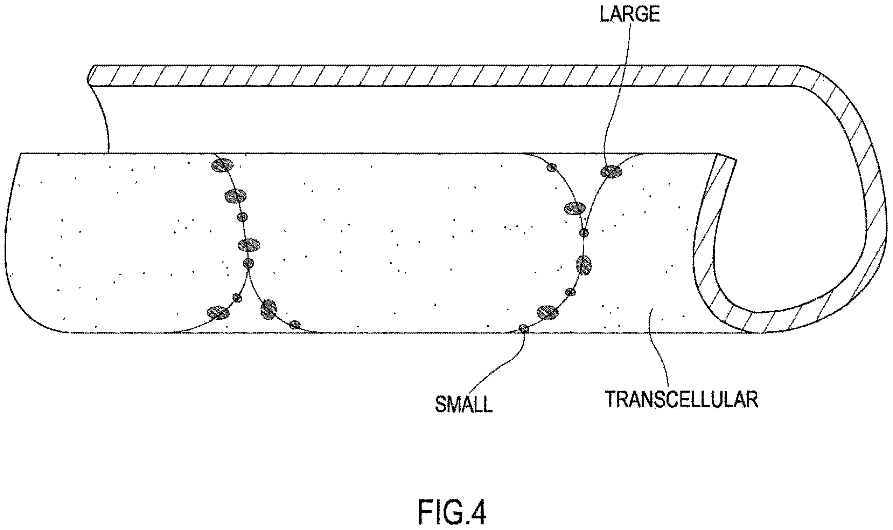

Referring now to FIG. 4, a cross-section from a peritoneal blood vessel illustrates the three-pores of the vessel.

The three-pores each have their own solute and UF clearance, leading to one kinetic model called the three-pore model. The three-pore model is a mathematical model that describes, correlates and predicts relationships among the time-course of solution removal, fluid transfer, treatment variables and physiological properties. The three-pore model is a predictive model that can be used for different types of treatment fluid, such as Dianeal®, Physioneal®, Nutrineal®, and Extraneal® dialysates marketed by Baxter.

The classical three-pore model (3PM) of peritoneal dialysis is more than 25 years old. Given what is known about the actual structure of the peritoneal barrier, the use of pores is phenomenological. However, the theory of hindered solute transport is most completely developed for the case of spherical solute molecules in a long cylindrical (or slit-shaped) pore while transport in other media is less completely understood. Hence, the 3PM represents an idealized 'equivalent barrier' which is used to describe the function of the peritoneal transport barrier rather than its structural or anatomical features. Separating the peritoneal membrane into, basically, three parallel porous membranes with different size-selective properties makes it possible to understand phenomena such as sodium sieving, middle-molecule transport and the osmotic behavior of polydisperse osmotic agents such as icodextrin. Since its first development, the 3PM has been modified numerous times. However, the classic 3PM does not simulate the inflow and/or outflow phase of the dwell. Notably, at higher dialysate flows, a significant part of the exchange time is spent either filling or draining the peritoneal cavity. Therefore, an extended 3PM having an additional compartment has been developed, allowing simulation also of the drain and fill phases of the dwell. The extended model may be then used to optimize the treatment with APD with regard to osmotic water transport (UF), small/middle-molecule clearance and glucose absorption. The results demonstrate that the 'metabolic cost' in terms of glucose absorption can be significantly reduced by using higher DFRs and a 'bimodal' regime where short dwells containing a high glucose concentration are combined with longer dwells containing no or a low glucose concentration. In addition, it is demonstrated that these regimes make it possible to shorten the total treatment time while achieving the same or better small-solute transport and UF.

**The modified 3-pore model differential equations**

During peritoneal dialysis, the net volume flow across the peritoneal membrane, at any time t from the start of the filling phase, is assumed to be the sum of six different volume flows:

$$\frac{dV_D}{dt} = J_{v,C} + J_{v,S} + J_{v,L} - L + J_{fill} - J_{drain} \tag{1}$$

In this equation, $J_{v,C}$, $J_{v,S}$, and $J_{v,L}$ represent the net flow of water (in mL/min) across the aquaporines, the highly selective pathways ("small pores") and the weakly selective pathways ("large pores"), respectively.

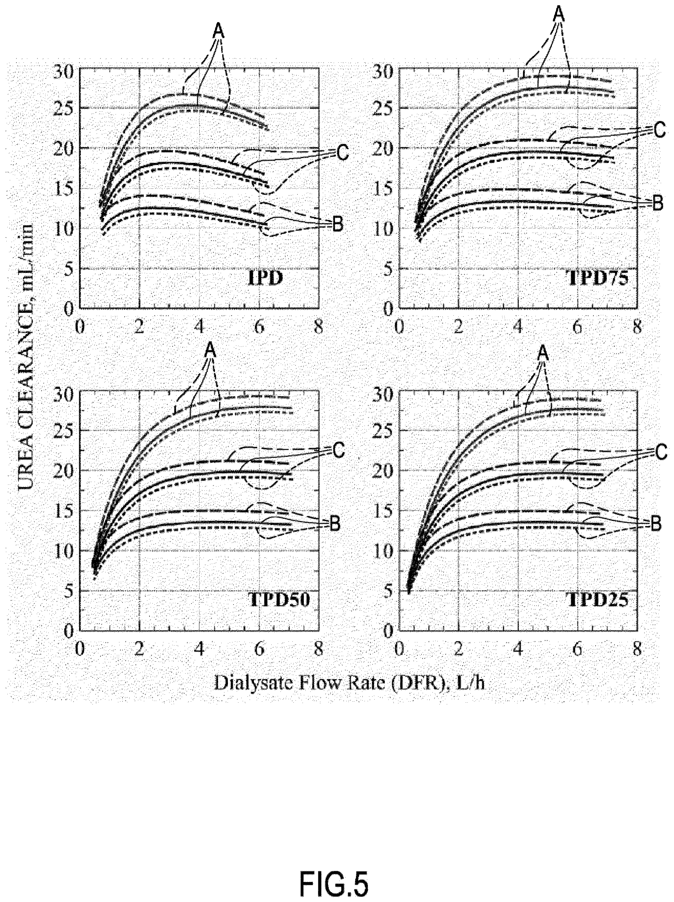

In other terms, $V_D$ is the peritoneal fluid volume, $J_{v,C}$ is the flow of fluid through transcellular pores or aquaporines shown in FIG. 5, $J_{v,S}$ is the flow of fluid through small pores shown in FIG. 5 and $J_{v,T}$ is the flow of fluid through large pores shown in FIG. 5. In the 3-pore model, the flows in equation 1 are assumed to vary only as a function of time and are directed into the peritoneal cavity when positive. The net lymphatic clearance from the peritoneal cavity to the circulation is denoted L (in mL/min) and is typically on the order of 0.2-0.3 mL/min when measured as a clearance to the circulation.

The model has been extended to include also the fill and drain phases of the dwell. Thus, $J_{drain}$ and $J_{fill}$ represent the flows of volume (in mL/min) to and from the source of treatment fluid, respectively. The change in the intra-peritoneal concentration of a solute i (denoted $dC_{D,i}/dt$ in mmol/mL/min) at any time t is dependent on three separate terms:

$$\frac{dC_{D,i}}{dt} = \frac{J_{s,S,i} + J_{s,L,i}}{V_D} - C_{D,i}\frac{J_{v,C} + J_{v,S} + J_{v,L} + J_{fill}}{V_D} + \frac{C_{B,i}J_{fill}}{V_D} \tag{2}$$

The first term $\left(\frac{J_{s,S,i} + J_{s,L,i}}{V_D}\right)$ is the change in intra-peritoneal concentration caused by the flow of solutes (through small and large pores, $J_{s,S,i}$ and $J_{s,L,i}$ in mmol/min) in and out of the peritoneal cavity. As can be seen, a positive solute flow is directed into the peritoneal cavity, increasing the concentration in the dialysate. The second term ( $C_{D,i}\frac{J_{v,C} + J_{v,S} + J_{v,L} + J_{fill}}{V_D}$ ) represents the dilution/concentration due to volume flux in and out of the peritoneum. Only water flows that affect the dialysate concentration are included in this term (i.e. L and $J_{drain}$ are not included). The last term ( $\frac{C_{B,i}J_{fill}}{V_D}$ ) is the change in concentration due to the inflow of fresh dialysate $J_{fill}$, having a concentration $C_{B,i}$ (in mmol/mL). The change in concentration in the drain reservoir of solute i ($dC_{B,i}(t)/dt$ in mmol/mL/min) is given by:

$$\frac{dC_{B,i}}{dt} = \frac{J_{drain}C_{D,i} - J_{fill}C_{B,i}}{V_B} - \frac{C_{B,i}}{V_B}\frac{dV_B}{dt} \tag{3}$$

The change in reservoir "bag" volume $V_B$ is simply

$$\frac{dV_B}{dt} = -J_{fill} + J_{drain} \tag{4}$$

Thus, the concentration in the reservoir does not change during the fill phase ($dC_{B,i}/dt=0$). This equation implies that the drain compartment is identical to the compartment with fresh dialysis fluid which is not the case in actual practice. However, since drain fluid and fresh dialysis fluid are never mixed, there is no need for more than one "reservoir" compartment in the model. Hence, the compartment $V_B$ acts as a source during the fill phase and as a collector of drain fluid during the drain phase. The initial conditions for the simulations are:

$$V_D(0) = V_r \tag{5}$$

$$C_{D,i}(0) = C_{p,i} \tag{6}$$

$$C_{B,i}(0) = C_{I,i} \tag{7}$$

$$V_B(0) = V_I \tag{8}$$

where $V_r$ is the residual volume, $V_I$ the fill/instilled volume (at the start of the fill phase) or 0 at the start of the drain phase), $C_{I,i}$ is the dialysis fluid concentration of solute i, $C_{p,i}$ is the plasma concentration of solute i which is assumed to be constant during the dwell. The ordinary differential equations (ODE) 1-4 above, along with the initial conditions, represent the initial value problem (IVP) to be solved in order to obtain the unknown functions $V_D(t)$, $C_{D,i}(t)$, $V_B(i)$ and $C_{B,i}(t)$.

**Volume and solute flow in the three-pore model**

The solute flow (in mmol/min) over each pathway is calculated according to the Patlak equation:

$$J_{s,S,i} = J_{v,S}(1 - \sigma_{s,i}) \frac{C_{P,i} - C_{D,i} e^{-Pe_{S,i}}}{1 - e^{-Pe_{S,i}}} \tag{9}$$

$$J_{s,L,i} = J_{v,L}(1 - \sigma_{L,i}) \frac{C_{P,i} - C_{D,i} e^{-Pe_{L,i}}}{1 - e^{-Pe_{L,i}}} \tag{10}$$

where $Pe_{S,i} = J_{vS}(1-\sigma_{S,i})/PS_{S,i}$ and $Pe_{L,i} = J_{vL}(1-\sigma_{L,i})/PS_{J,i}$ are the Péclet numbers (the ratio between the maximum convective and diffusive clearance for solute i) for the small and large pore pathway, respectively. The mass transfer area coefficients, $PS_{S,i}$ and $PS_{L,i}$ (in mL/min), are either set according to following Table 1 or calculated according to pore theory $PS = D \cdot A_0/\Delta x \cdot A/A_0$ where $A/A_0$ is the diffusive restriction factor and D is the free diffusion coefficient. The reflection coefficients are calculated according to theory. The volume flow (mL/min) is calculated using Starling equilibria over each parallel pathway

$$J_{vC} = \alpha_C L_p S\left(\Delta P - RT \sum_{i=1}^{N} \varphi_i \left(C_{p,i} - C_{D,i}\right)\right) \tag{11}$$

$$J_{vS} = \alpha_S L_p S\left(\Delta P - RT \sum_{i=1}^{N} \varphi_i \sigma_{S,i}(C_{p,i} - C_{D,i})\right) \tag{12}$$

$$J_{vL} = \alpha_L L_p S\left(\Delta P - RT \sum_{i=1}^{N} \varphi_i \sigma_{L,i}(C_{p,i} - C_{D,i})\right) \tag{13}$$

where $\alpha_C$, $\alpha_S$ and $\alpha_L$ are the fractional hydraulic conductances for the different pathways (see next Table 1), $\varphi_i$ is the osmotic coefficient for solute i, R is the gas constant and T is the body temperature (310°K). Thus, the osmotic reflection coefficients are assumed to be the same for osmosis and solute transport. To account for the recruitment/loss of peritoneal surface area due to a high/low IPV, an area factor was multiplied to all PS-values and $L_p S$ according to Keshavia et al (Keshaviah P, Emerson PF, Vonesh EF, and Brandes JC. Relationship between body size, fill volume, and mass transfer area coefficient in peritoneal dialysis. J Am Soc Nephrol 4: 1820-1826, 1994.).

$$af = 16.18(1 - e^{-0.00077 \cdot V_D(t)})/13.3187 \qquad (14)$$

Thus, the mass transfer area coefficients and the filtration coefficient were inflated for volumes > 2250 mL and vice versa.

**Pressure dynamics in the 3-pore model**

The average capillary hydrostatic pressure was calculated according to a pre-to-post-capillary resistance ratio (PTP=$R_a/R_v$) of 8:1. Thus, given the mean arterial pressure (MAP) and the large-vein pressure ($P_v$) of the patient, the capillary pressure is calculated according to the equation:

$$P_c = fR_v \cdot MAP + fR_a \cdot P_v \qquad (15)$$

where $fR_a$=1-$fR_v$ and $fR_a$=PTP/(PTP+1) are the fractional pre-capillary and post-capillary resistances, respectively. The net hydrostatic pressure gradient is simply

$$\Delta P = P_c - IPP \qquad (16)$$

where the intra-peritoneal pressure (IPP) was assumed to be dependent only on the intraperitoneal volume (IPV). A modified equation by Twardowski et al for the supine position is used (Twardowski ZJ, Prowant BF, Nolph KD, Martinez AJ, and Lampton LM. High volume, low frequency continuous ambulatory peritoneal dialysis. Kidney Int 23: 64-70, 1983):

$$IPP = 4.7 + \frac{VD(t)}{690} \qquad (17)$$

The intercept used is higher than that obtained in the study by Twardowski et al since the IPP for a total IPV of 2250 mL is here assumed to be 8 mmHg in the supine position. However, in a patient naive to peritoneal dialysis, a lower (negative) intercept can be expected. Furthermore, IPP might not be a completely linear function of IPV especially in the lower and higher ranges. In the current article, a MAP of 90 mmHg was used and, further, it is assumed that the large-vein pressure is equal to the intra-peritoneal pressure ($P_v$=IPP).

**Temporal discretization and numerical solution of the 3-pore IVP**

To solve above equations (1-4) numerically, a fourth order Runge-Kutta scheme was implemented. If N represents the total number of solutes included in the simulation, the system of equations (1-4) can be re-written:

$$\frac{dV_D}{dt} = \mathcal{F}(t, C_{D,1}, C_{D,2}, \ldots, C_{D,N}) \qquad (18)$$

$$\frac{dC_{D,i}}{dt} = \mathcal{G}(t, C_{D,i}, C_{B,i}, V_D) \qquad (19)$$

$$\frac{dC_{B,i}}{dt} = \mathcal{H}(t, C_{D,i}, C_{B,i}, V_B) \qquad (20)$$

where $\mathcal{F}$, $\mathcal{G}$ and $\mathcal{H}$ are functionals ("functions of functions") corresponding to the right-hand side of equations 1-3 and i=0,1, ..., N. All functions of interest ($V_D(t)$, $C_{D,i}(t)$, $V_B(i)$ and $C_{B,i}(t)$) are defined on Q+1 equally spaced grid points over the total simulation time [0,$\tau$] with a time-step $\Delta$t=$\tau$/Q. The grid points are labeled k=0,1,2, ..., Q with k=0 representing the initial values. The functions are then calculated on the grid points $C_{D,i,k}$=$C_{D,i}$(k$\Delta$t), $C_{B,i,k}$=$C_{B,i}$(k$\Delta$t) and $V_{D,i,k}$= $V_{D,i}$(k$\Delta$t). Starting with the initial value (k=0) the next grid point (k+1) is calculated according to:

$$K_1 = \Delta t \cdot \mathcal{F}(k\Delta t, C_{D,1,k}, C_{D,2,k}, \ldots, C_{D,N,k}) \qquad (21)$$

$$L_{1,i} = \Delta t \cdot \mathcal{G}(k\Delta t, C_{D,i,k}, C_{B,i,k}, V_{D,k}) \tag{22}$$

$$M_{1,i} = \Delta t \cdot \mathcal{H}(k\Delta t, C_{D,i,k}, C_{B,i,k}, V_{D,k}) \tag{23}$$

$$K_2 = \Delta t \cdot \mathcal{F}(k\Delta t + \tfrac{\Delta t}{2}, C_{D,1,k} + \tfrac{L_{1,1}}{2}, C_{D,2,k} + \tfrac{L_{1,2}}{2}, \dots, C_{D,N,k} + \tfrac{L_{1,N}}{2}) \tag{24}$$

$$L_{2,i} = \Delta t \cdot \mathcal{G}(k\Delta t + \tfrac{\Delta t}{2}, C_{D,i,k} + \tfrac{L_{1,i}}{2}, C_{B,i,k} + \tfrac{M_{1,i}}{2}, V_{D,k} + \tfrac{K_1}{2}) \tag{25}$$

$$M_{2,i} = \Delta t \cdot \mathcal{H}(k\Delta t + \tfrac{\Delta t}{2}, C_{D,i,k} + \tfrac{L_{1,i}}{2}, C_{B,i,k} + \tfrac{M_{1,i}}{2}, V_{D,k} + \tfrac{K_1}{2}) \tag{26}$$

$$K_3 = \Delta t \cdot \mathcal{F}(k\Delta t + \tfrac{\Delta t}{2}, C_{D,1,k} + \tfrac{L_{2,1}}{2}, C_{D,2,k} + \tfrac{L_{2,2}}{2}, \dots, C_{D,N,k} + \tfrac{L_{2,N}}{2}) \tag{27}$$

$$L_{3,i} = \Delta t \cdot \mathcal{G}(k\Delta t + \tfrac{\Delta t}{2}, C_{D,i,k} + \tfrac{L_{2,i}}{2}, C_{B,i,k} + \tfrac{M_{2,i}}{2}, V_{D,k} + \tfrac{K_2}{2}) \tag{28}$$

$$M_{3,i} = \Delta t \cdot \mathcal{H}(k\Delta t + \tfrac{\Delta t}{2}, C_{D,i,k} + \tfrac{L_{2,i}}{2}, C_{B,i,k} + \tfrac{M_{2,i}}{2}, V_{D,k} + \tfrac{K_2}{2}) \tag{29}$$

$$K_4 = \Delta t \cdot \mathcal{F}(k\Delta t + \Delta t, C_{D,1,k} + L_{3,1}, C_{D,2,k} + L_{3,2}, \dots, C_{D,N,k} + L_{3,N}) \tag{30}$$

$$L_{4,i} = \Delta t \cdot \mathcal{G}(k\Delta t + \Delta t, C_{D,i,k} + L_{3,i}, C_{B,i,k} + M_{3,i}, V_{D,k} + K_3) \tag{31}$$

$$M_{4,i} = \Delta t \cdot \mathcal{H}(k\Delta t + \Delta t, C_{D,i,k} + L_{3,i}, C_{B,i,k} + M_{3,i}, V_{D,k} + K_3) \tag{32}$$

$$V_{D,k+1} = V_{D,k} + \frac{K_1 + 2K_2 + 2K_3 + K_4}{6} \tag{33}$$

$$C_{D,i,k+1} = C_{D,i,k} + \frac{L_{1,i} + 2L_{2,i} + 2L_{3,i} + L_{4,i}}{6} \tag{34}$$

$$C_{B,i,k+1} = C_{B,i,k} + \frac{M_{1,i} + 2M_{2,i} + 2M_{3,i} + M_{4,i}}{6} \tag{35}$$

$$V_{B,i,k+1} = V_{B,i,k} + \Delta t(J_{drain} - J_{fill}) \tag{36}$$

The total simulation time ($\tau$) was chosen so that a total of 25 subsequent dwells was simulated with a time step of 0.001 min. This short time step was chosen so that the error in fill/drained volume would be less than 0.1 mL per dwell.

**Regulation of fill/drain cycles**

In the simulations, a fill flow rate of 200 mL/min was used. For the drain phase, drain flow rates of 350 mL/min (fast-phase) and 36 mL/min (slow-phase) were implemented with a transition point (break point) at an intraperitoneal volume of 381 mL. Each cycle starts with a fill phase followed by a dwell phase which lasts for a pre-determined dwell time (DT) after which the drain phase starts. The whole duration of the cycle, consisting of the fill-, dwell- and drain-time is referred to as the exchange time (ET). For IPD, the drain phase ends when the calculated intra-peritoneal volume for the next grid point is less than the residual volume ($V_{D,k+1} < Vr$) after which either a new cycle starts or the simulation ends. Depending on the time-step chosen, this leads to a small error since the actual volume left in the peritoneal cavity after a cycle will always be larger than (or equal to) $V_r$. For TPD without overfill or overdrain, the drain phase terminates when the calculated volume for the next grid point is less than the sum of the residual volume and the tidal reserve volume ($V_{D,k+1} < V_r + TRV$).

Moreover, IPD was simulated for fill and drain volumes of 2 L, while TPD was simulated using a tidal volumes of 0.5L, 1 L, or 1.5L with full drains and subsequent fills (2L) occurring after every fifth dwell. A total of 25 cycles for a large number of different DFRs were simulated using 3 different glucose concentrations (1.5%, 2.27% and 3.86%) and 3 different peritoneal transport types: low (PET $D/P_{crea} < 0.6$), high (PET $D/P_{crea} > 0.8$) and average.

**Table 1 -** Parameters used for computer simulations of intraperitoneal volume vs. time V(t) curves according to a three-pore model of membrane selectivity

| | |
|---|---|
| Small pore radius ($r_s$) (Å) | 43 |
| Large pore radius ($r_L$) (Å) | 250 |
| Fractional small pore UF-coeff. ($\alpha_s$) | 0.900 |
| Fractional transcellular UF-coeff. ($\alpha_c$) | 0.020 |
| Fractional large pore UF-coeff. ($\alpha_L$) | 0.080 |
| Ultrafiltration coefficient ($L_pS$)(mL/min/mmHg) | 0.074 |
| Osmotic conductance to glucose ($L_pS\,\sigma_g$) ($\mu$L/min/mmHg) | 3.6 |
| "Unrestricted" pore area over unit diffusion distance for small pores ($A_0/\Delta X)_s$(cm) | 25,000* |
| PS ("MTAC") for glucose (mL/min) | 15.4 |
| PS ("MTAC") for urea (mL/min) | 26.0 |
| PS ("MTAC") for "Na" and "anion" (mL/min) | 4.5 |
| PS ("MTAC") for phosphate (mL/min) | 10.2 |
| Peritoneal lymph flow (L)(mL/min) | 0.3 |
| Transperitoneal oncotic pressure gradient ($\Delta\pi_{prot}$) (mmHg) | 22 |
| Peritoneal residual volume ($V_r$) (mL) | 250 |
| Serum urea concentration (mmol/L) | 20 |
| Serum creatinine concentration ($\mu$mol/l) | 660 |
| Dialysis fluid sodium concentration (mmol/L) | 132 |
| Serum sodium (and sodium associated "anion" concentration) (mmol/L) | 140 |
| Serum glucose concentration (mmol/L) | 6.5 |
| Dissociation factor for "Na$^+$" and "anions" | 0.93 |
| *25,000 cm was used for an average peritoneal transport type, 40,000 cm for high transporters and 15,000 cm for low transporters. | |

**Results from simulations**

*Urea clearance*

In Figure 5, the simulated urea clearance as a function of DFR is plotted for the different techniques (IPD, TPD75/50/25) and different transport types: Fast (lines labeled A), Average (lines labeled C), Slow (lines labeled B) for three different glucose concentrations: 1.5% (dotted line), 2.27% (solid line) and 3.86% (dashed line). At low to moderate dialysate flow rates (<2-3L/h) the intermittent technique provides slightly higher clearances than the tidal technique. For slow transporters, higher volume flows become ineffective (reach a plateau) at lower DFRs compared to average and fast transport types. Thus, for small solute transport, there is little benefit in exceeding 2L/h for a slow transporter. For the lower tidal volumes (TPD50 and TPD25), the urea clearance is lower in the leftmost part of the curve compared to the other modalities, demonstrating a relative inefficiency of low tidal volumes at lower DFRs. The right-most value for each curve represents the maximal flow rate possible at the chosen fill and drain flow rates (i.e. all time is spent either filling or draining the peritoneal cavity) and is, expectedly, higher for the tidal technique. The results for the other small solutes, creatinine, sodium and phosphate are very similar to the urea results (data not shown) although the transport of sodium more closely follow the UF curve.

*Osmotic water transport (UF)*

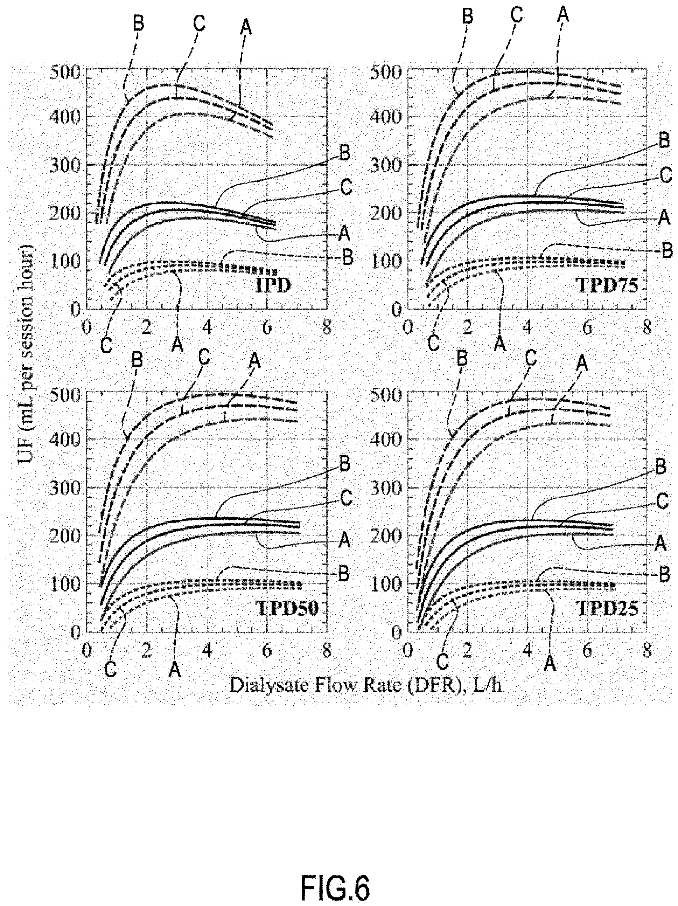

In Figure 6, the osmotic water transport, or "UF", per session hour is shown as a function of DFR. Expectedly, in absolute terms, the UF is higher for the slow transporters due to the slower dissipation of glucose, improving the average osmotic pressure gradient. The peak values occur at similar DFRs compared to the urea clearance vs. DFR curves in Fig. 6. At first glance, this might seem a bit surprising, since it is at these DFRs that the glucose absorption is at its greatest. However, the increased glucose dissipation at these high DFRs will be more than well compensated by the influx of fresh dialysis fluid. Thus the glucose gradient will be maintained despite increasing absorption. Thus, it is the addition of fresh dialysis fluid that will increase both UF and clearance of small solutes at higher DFRs. The inefficiency at higher flows is due to the fact that, in relative terms, more time is spent filling and draining the peritoneal cavity, leading to a decrease in both UF and small solute clearance.

*Osmotic water transport (UF) efficiency*

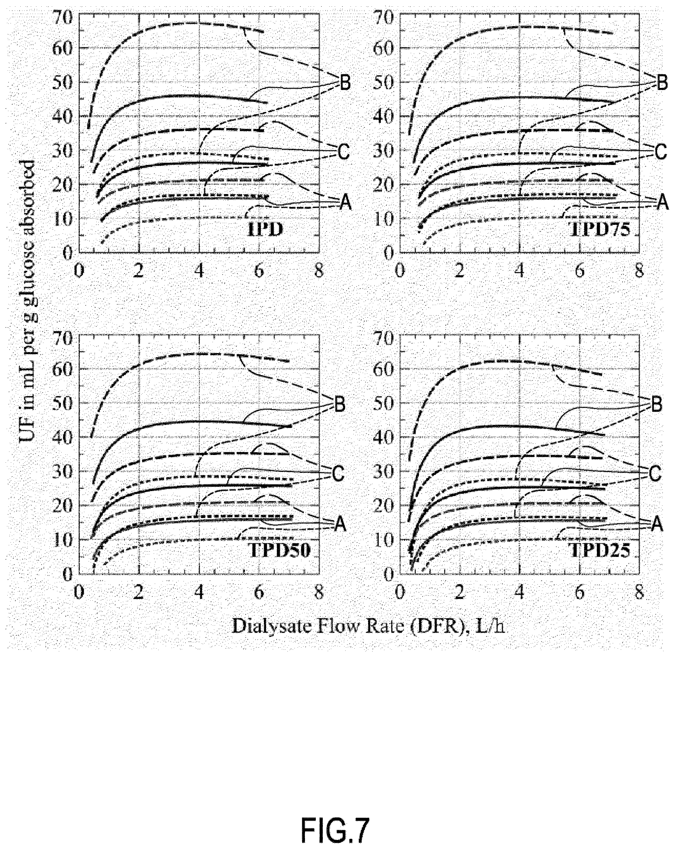

The osmotic transport of water (in mL) occurs at a "metabolic cost", in terms of glucose absorption. In Figure 7 the UF in mL per gram glucose absorbed (or "UF efficiency") is plotted as a function of DFR. The UF efficiency is markedly improved by increasing the DFR up to about 2L/h after which a plateau is reached and small or no further improvements are attained. The higher glucose concentrations are far more efficient in achieving UF. Thus, at a DFR of 2L/h, the patient will absorb more than twice the amount of glucose for the same amount of UF using the 1.5% solution compared to the 3.86% solution.

*Small-solute transport efficiency (mmol UreaR per g glucose absorbed)*

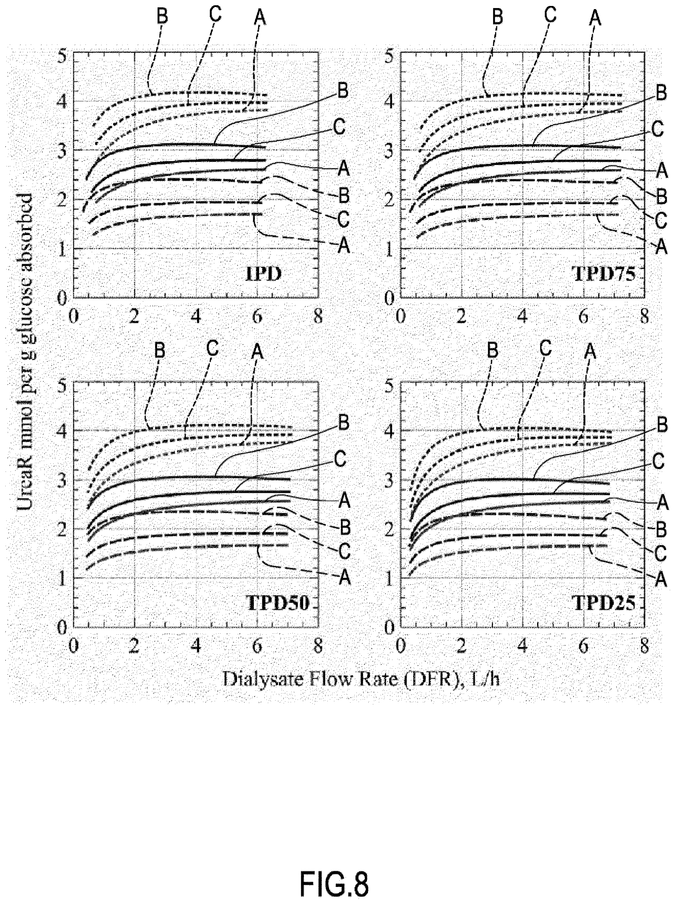

In Figure 8, the small solute transport "efficiency" (in mmol urea removed per g glucose absorbed) as a function of DFR is plotted. Similar to the osmotic efficiency, the removal reaches an early plateau at DFRs higher than 2L/h. However, here the situation is the opposite compared to UF efficiency. The higher glucose concentrations are much less efficient in achieving urea transport. Similarly, the patient will absorb almost twice the amount of glucose per mmol of urea removed using a 3.86% solution instead of a 1.5% solution.

*Middle molecule transport vs. DFR*

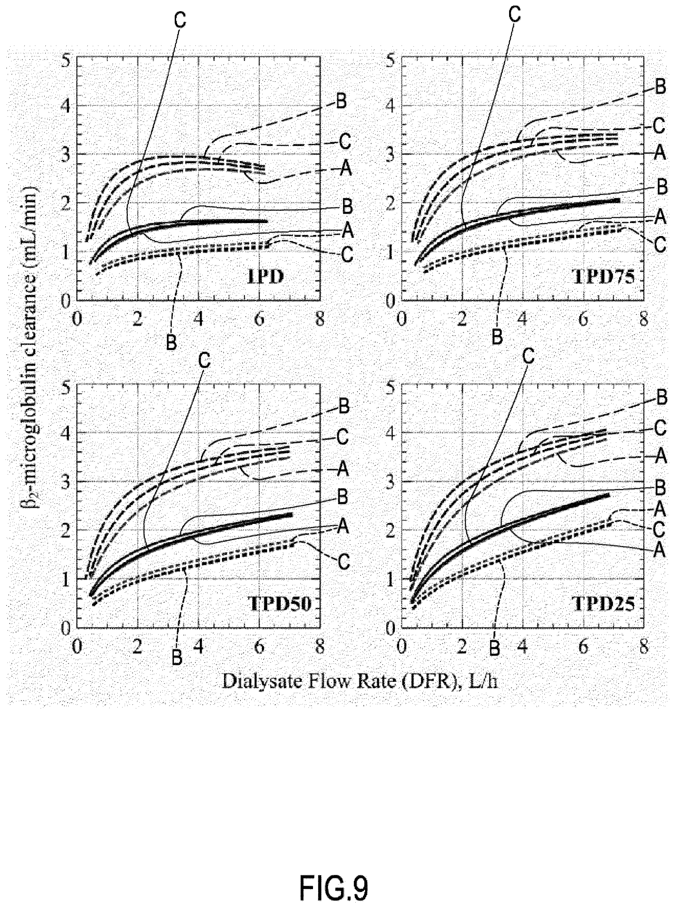

In Figure 9, the clearance of $\beta_2$-microglobulin as a function of DFR is shown. In comparison to the results for the small-solute transport, no peak or decrease in clearance was observed at higher DFRs for the tidal techniques. Furthermore, smaller tidal volumes are clearly beneficial for middle-molecule transport.

*Comparison with clinical studies*

In Table 2, the extended 3PM is compared with the study by Aasaröd and colleagues. There is good agreement between the model and the clinical measurements, although there seems to be a tendency for the model to underestimate the clearances at higher DFRs.

Table 2

| DFR | IPD $Cl_{urea}$ | TPD 50% $Cl_{urea}$ |
|---|---|---|
| 1.1 L/h | 14.3* mL/min (14.9) | 13.3 mL/min (13.9) |
| 1.6 L/h | 16.9 mL/min (17.0) | 15.9 mL/min (16.2) |
| 2.7 L/h | 20.9 mL/min (18.8) | 19.9 mL/min (19.1) |

*Osmotic efficiency*

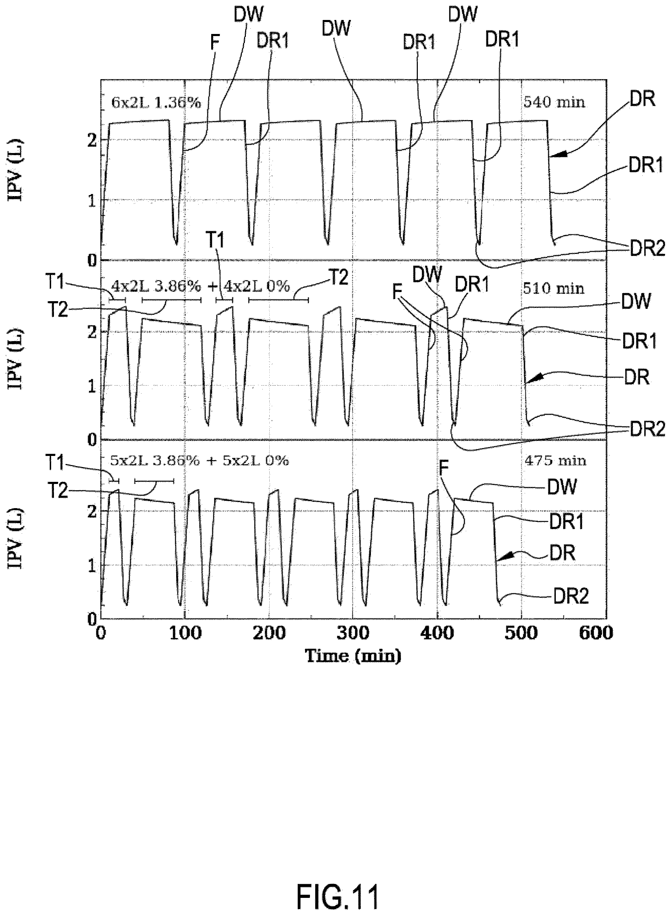

The osmotic efficiency expressed in terms of UF per liter dialysis fluid "consumed" as a function of DFR is shown in Figure 11 for the different techniques, transport types and different glucose concentrations. Generally, the osmotic efficiency is markedly improved by increasing the DFR up to about 2L/h after which a plateau is reached and small or no further improvements can be observed.

*Optimization Procedure*

According to the above discussed results, if the osmotic agent (e.g. glucose) strength is varied and low or no osmotic agent strengths (preferably <0.5% glucose) during some of the cycles, while the osmotic agent strengths are kept high in other cycles (preferably >3.86% glucose), it is possible to separate volume removal from the small solute removal and thereby maximize them both and at the same time minimize the osmotic agent (e.g. glucose) taken up by the patient.

In more general terms, the treatment procedure executed by the control system 74 comprises a sequence of cycles alternating in a proper arrangement high glucose strength treatment fluid with low or no glucose strength treatment fluids. In the present description alternating high osmotic agent strength treatment fluid with low or no osmotic agent strength treatment fluid, does not imply that a cycle with high osmotic agent strength treatment fluid is immediately followed by a cycle with low or no osmotic agent strength treatment fluid and vice versa; indeed, intermediate additional cycles may be included between the mentioned high and low/no osmotic agent cycles.

The control system 74 of the PD apparatus is programmed to run a fill phase of a first cycle delivering a first treatment fluid to the patient, in which the first treatment fluid has a first concentration of the osmotic agent; subsequently to the first cycle, the control system 74 runs a fill phase of a second cycle delivering a second treatment fluid to the patient, in which the second treatment fluid has a second concentration of the osmotic agent different from the concentration of the first treatment fluid; subsequently to the second cycle, the control system 74 runs a fill phase of a third cycle delivering a third treatment fluid to the patient, in which the third treatment fluid has a third concentration of the osmotic agent different from the concentration of the second treatment fluid.

In the sequence, the second concentration of the osmotic agent is higher than the first concentration of the osmotic agent and the third concentration of osmotic agent is lower than the second concentration of osmotic agent (the first and the third concentration of osmotic agent may be or may be not identical).

Alternatively, in the mentioned sequence, the second concentration of the osmotic agent is lower than the first concentration of the osmotic agent and the third concentration of osmotic agent is higher than the second concentration of osmotic agent (the first and the third concentration of osmotic agent may be or may be not identical).

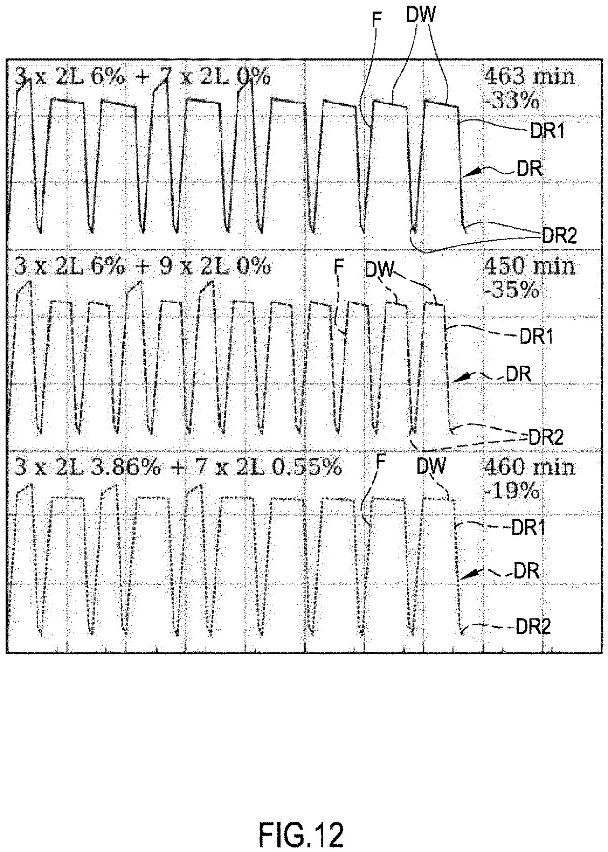

It is noted that first, second and third cycles do not imply that they are executed one immediately after the other. In other terms, the first cycle may be followed by one or more additional cycles before running the second cycle and/or the second cycle may be followed by one or more additional cycles before running the third cycle. In other terms, first, second and third cycle only imply that there is a temporal sequence and thereby the first cycle is executed before the second cycle, which, in turn, is executed before the third cycle during a treatment session. In the example of figure 12, upper treatment session, the first cycle with 6% glucose concentration is immediately followed by the second cycle with 0% glucose concentration; then an additional cycle with 0% glucose concentration is performed before the third cycle, again with 6% glucose concentration is run.

Generalizing the above concept, the control system 74 delivers a plurality of cycles using a treatment fluid with high concentration of osmotic agent, alternating with a plurality of cycles using a treatment fluid with low or no concentration of osmotic agent. Again and as previously explained, there may be a certain number of cycles with high glucose concentration followed by a certain number of cycles with low/no glucose concentration before a new cycle with high glucose concentration.

The treatment fluid with low concentration of osmotic agent includes a concentration of osmotic agent equal or lower than 2,5% in weight, optionally equal or lower than 2% in weight, optionally equal or lower than 1,5% in weight, and in more detail equal or lower than 0,5% in weight. The treatment fluid with low osmotic agent/glucose concentration also includes a treatment fluid with no osmotic agent/glucose.

The treatment fluid with high concentration of osmotic agent includes a concentration of osmotic agent equal or higher than 2,5% in weight, optionally equal or higher than 3% in weight, and in more detail equal or higher than 3,86% in weight. In certain treatment sessions also an osmotic agent with a concentration higher than 5% strength may be used.

Additionally, it is relevant to mention that the procedure includes leaving the treatment fluid in the peritoneal cavity for a dwell time, wherein the dwell time of a treatment fluid with high osmotic agent concentration is different from, and in particular lower than, a dwell time of a treatment fluid with low or no osmotic agent concentration.

In other terms, the dwell time of the treatment fluid having high glucose strength is much shorter that the dwell time of the treatment fluid with low/no glucose strength.

As can be immediately inferred, in case an apparatus according to figure 1 is used, at least two sources S (or bags 6) of treatment fluid have a different content of osmotic agent (one will have a high glucose strength and the other will have a low/no glucose strength). The two sources are properly alternated in the cycles as above described up to the end of the treatment session.

As also explained, starting from a discrete number of bags with different osmotic agent concentration, it is possible to generate blended solution with an intermediate glucose concentration to better fit with the patient needs.

Using, vice versa, the apparatus of figure 2, the concentrates may be properly mixed to obtain the treatment fluid with the correct osmotic agent concentration to be used for the specific cycle/cycles; then a different treatment fluid with different osmotic agent concentration is generated to administer the other cycle/cycles in the correct alternating treatment session.

A similar procedure is adopted with the apparatus of figure 3, properly mixing the osmotic agent concentrate solution with the dialysis solution.

The control system 74 may receive the prescription for the treatment session (e.g. night session) in terms of number of cycles for the treatment session, as well as in terms of concentration of osmotic agent and dwell time for each cycle of the treatment session.

Alternatively, the control system 74 includes a memory storing a prediction algorithm; the control system 74 receives as input the desired total UF volume to be removed at the end of the treatment session and a desired total small solute volume, e.g. urea volume, to be removed at the end of said treatment session and, based on said prediction algorithm, is configured to determine at least the first concentration of osmotic agent in the first treatment fluid, the second concentration of osmotic agent in the second treatment fluid and the third concentration of osmotic agent in the third treatment fluid. In general, based on the prediction algorithm, the control system may determine the concentration of osmotic agent and the dwell time of each of the cycles of the treatment session; eventually the prediction algorithm may also indicate the optimum number of cycles in the treatment session. The prediction algorithm may be based on the modified three pore model equation (1) .

*Optimization Examples*

In Figures 11 and 12, some possible optimization examples are shown.

Figure 11, upper graphic shows a "standard prescription" for peritoneal dialysis of 6x2L 1.36% glucose with a duration of 9 hours (540 min). The treatment session consists of 6 identical cycles and is a night session.

Each of the fill phases F (with a treatment fluid having 1,36% strength in glucose) fills the patient peritoneal cavity up to a bit more than 2 liters of treatment fluid.

During each dwell phase DW the fluid volume in the peritoneal cavity increases since fluids moves from the patient body to the peritoneal cavity.

The drain phase DR is then shown having two slopes $DR_1$, $DR_2$ corresponding to the initial fast drain step and to the final slow drain step.

Six identical cycles in the same treatment session are shown having substantially the same shape (same effects).

The corresponding transport parameters in terms of urea removal, UF, glucose absorption are shown in Table 3, first line.

The upper curve is compared with scenarios were each dwell is optimized for either UF (using 3.86% glucose) and/or small-solute transport (using 0% glucose) keeping the glucose absorption low. The treatment time for the two latter scenarios was chosen to fit the UF and urea transport of the "standard prescription", i.e. same UF and same urea removal targets.

The intermediate curve includes 8 cycles of two different alternating treatment fluids.

The first cycle is performed with a high glucose concentration treatment fluid (3,86% strength) and has a short dwell time $T_1$. Each cycle has a fill phase F, a dwell phase DW and a drain phase DR with first fast drain $DR_1$ and second slow drain $DR_2$.

In the first cycle, during the dwell phase, the volume inside the peritoneal cavity increases due to net fluid transfer towards the peritoneal cavity.

The second cycle is performed with a low/no glucose concentration treatment fluid (0% strength) and has a longer dwell time $T_2$.

In the second cycle, during the dwell phase, the volume inside the peritoneal cavity decreases due to net fluid transfer from the peritoneal cavity towards the patient body.

The third and the fifth cycles are identical to the first cycle, while the fourth and the sixth cycles are identical to the second cycle.

As apparent from below table 3, the urea removal and the UF of the upper and intermediate treatment sessions are essentially the same.

Vice versa, the glucose absorption is reduced by about 19% and the total treatment time for achieving the mentioned target removals is reduced to 510 min.

The lower treatment session again optimize UF, urea removal, treatment time and glucose absorption.

The treatment session is made of 10 cycles; the first cycle is performed with a high glucose concentration treatment fluid (3,86% strength) and has a short dwell time $T_1$ (shorter than in the previously described first cycle of the intermediate session).

In the first cycle, during the dwell phase, the volume inside the peritoneal cavity increases due to net fluid transfer towards the peritoneal cavity.

The second cycle is performed with a low/no glucose concentration treatment fluid (0% strength) and has a dwell time $T_2$ longer then the dwell time $T_1$.

In the second cycle, during the dwell phase, the volume inside the peritoneal cavity decreases due to net fluid transfer from the peritoneal cavity towards the patient body.

The third, the fifth and the seventh cycles are identical to the first cycle, while the fourth, the sixth and the eighth cycles are identical to the second cycle.

As apparent from below table 3, the urea removal and the UF of the upper and lower treatment sessions are essentially the same.

Vice versa, the glucose absorption is reduced by about 22% and the total treatment time for achieving the mentioned target removals is reduced to 475 min.

This clearly reflects in a benefit for the patient.

Table 3

| Regime | UreaR | UF | Glucose abs. | Decrease | Total time |
|---|---|---|---|---|---|
| 6x2L 1.36% | 158 mmol | 458 mL | 41.5 g | 0 % | 540 min |
| 4x2L 3.86% + 4x2L 0% | 158 mmol | 456 mL | 33.8 g | -19% | 510 min |
| 5x2L 3.86% + 5x2L 0% | 157 mmol | 457 mL | 32.3 g | -22% | 475 min |

Figure 12 shows other possible optimization embodiments.

The upper treatment session includes 10 cycles. The first cycle uses a treatment fluid with high glucose concentration (60 strength) for a short time $T_1$.

The second and third cycles use a treatment fluid with no glucose content. Dwell time $T_2$ is longer and net fluid is transferred to the patient.

The treatment session then provides a fourth and a sixth cycle with high glucose content and a fifth, seventh, eighth, ninth and tenth cycle with no glucose.

Again total treatment time is reduced to 463 min and targets in UF and urea removal are unaltered with respect to table 3.

Intermediate graph in figure 12 illustrates a treatment session of 12 cycles, the first, fourth and sixth make use of a treatment fluid having a glucose concentration of 6%. All the other nine cycles have no glucose in the treatment fluid.

Total treatment time is reduced to 450 min and targets in UF and urea removal are unaltered with respect to table 3.

A final example is presented in the lower portion of fig. 12. Ten cycles are performed by the cycler. First, third and fifth cycles have a treatment fluid with high glucose content (3,86%), the other seven cycles are performed with a treatment fluid having a low glucose content of 0,55%.

Total treatment time is reduced to 460 min and targets in UF and urea removal are unaltered with respect to table 3.

Notwithstanding the fact that all the examples make use of two different treatment fluids only, it is possible to use three of more treatment fluids differing each other due to the osmotic agent concentration, e.g. a first cycle with 6% glucose concentration, a second cycle with no glucose, a third cycle with 3,86% glucose concentration, a fourth cycle with 0,55% glucose concentration, etc.

Certain aspects of the invention are further described in the following numbered paragraphs.

Numbered Paragraph 1. An apparatus for peritoneal dialysis (1) comprising:

- an automated peritoneal dialysis cycler (3) programmed to run a treatment session including a plurality of cycles, each cycle including a fill phase, a dwell phase, and a drain phase of a peritoneal cavity of a patient (P), the treatment session lasting at most 720 min, the cycler (3) having:

- a patient line (54) configured to be placed in communication with the peritoneal cavity of the patient (P);
- at least a source (S) of treatment fluid which includes an osmotic agent;
- at least one pump (5) to circulate the treatment fluid to be delivered to the patient through said patient line (54);

- a control system (74) configured to drive said cycler (3) to deliver the treatment session, wherein, during the same treatment session, the control system (74) is programmed to:

> run a fill phase of a first cycle delivering a first treatment fluid to the patient, the first treatment fluid having a first concentration of the osmotic agent;

> subsequently to the first cycle, run a fill phase of a second cycle delivering a second treatment fluid to the patient, the second treatment fluid having a second concentration of the osmotic agent different from the concentration of the first treatment fluid; and

> subsequently to the second cycle, run a fill phase of a third cycle delivering a third treatment fluid to the patient, the third treatment fluid having a third concentration of the osmotic agent different from the concentration of the second treatment fluid;

wherein the second concentration of the osmotic agent is lower than the first concentration of the osmotic agent and the third concentration of osmotic agent is higher than the second concentration of osmotic agent, or wherein the second concentration of the osmotic agent is higher than the first concentration of the osmotic agent and the third concentration of osmotic agent is lower than the second concentration of osmotic agent.

Numbered Paragraph 2. Apparatus according to the previous numbered paragraph, wherein the osmotic agent is, or include, glucose, L-carnitine, glycerol, or combinations thereof, in particular the osmotic agent being or including glucose.

Numbered Paragraph 3. Apparatus according to any one of the previous numbered paragraphs, wherein the control system (74) is programmed to run the first cycle followed by one or more additional cycles before running the second cycle and/or wherein the control system (74) is programmed to run the second cycle followed by one or more additional cycles before running the third cycle.

Numbered Paragraph 4. Apparatus according to any one of the previous numbered paragraphs 1 or 2, wherein the control system (74) is programmed to run the first cycle immediately followed by the second cycle and/or wherein the control system (74) is programmed to run the second cycle immediately followed by the third cycle.

Numbered Paragraph 5. Apparatus according to any one of the previous numbered paragraphs, wherein, during said same treatment session, the control system (74) delivers a plurality of cycles using a treatment fluid with high concentration of osmotic agent, alternating with a plurality of cycles using a treatment fluid with low or no concentration of osmotic agent, said high concentration of osmotic agent being higher than said low concentration of osmotic agent.

Numbered Paragraph 6. Apparatus according to the previous numbered paragraph, wherein the treatment fluid with high concentration of osmotic agent includes a concentration of osmotic agent equal or higher than 3% by weight, optionally equal or higher than 4% by weight, and in more detail equal or higher than 5% by weight, said osmotic agent being in particular glucose.

Numbered Paragraph 7. Apparatus according to any one of the previous numbered paragraphs 5 or 6, wherein the treatment fluid with low concentration of osmotic agent includes a concentration of osmotic agent equal or lower than 2% by weight, optionally equal or lower than 1.5% by weight, and in more detail equal or lower than 0.5% by weight, said osmotic agent being in particular glucose.

Numbered Paragraph 8. Apparatus according to any one of the previous numbered paragraphs 5 to 7, wherein in said plurality of cycles with high concentration of osmotic agent alternating with said plurality of cycles with low or no concentration of osmotic agent, the high concentration of osmotic agent cycle is followed by one or more subsequent cycles before the low or no concentration of osmotic agent cycle.

Numbered Paragraph 9. Apparatus according to any one of the previous numbered paragraphs 5 to 8, wherein in said plurality of cycles with high concentration of osmotic agent alternating with said plurality of cycles with low or no concentration of osmotic agent, the low or no concentration of osmotic agent cycle is followed by one or more subsequent cycles before the high concentration of osmotic agent cycle.

Numbered Paragraph 10. Apparatus according to any one of the previous numbered paragraphs 5 to 7, wherein in said plurality of cycles with high concentration of osmotic agent alternating with said plurality of cycles with low or no concentration of osmotic agent, the high concentration of osmotic agent cycle is immediately followed by the low or no concentration of osmotic agent cycle.

Numbered Paragraph 11. Apparatus according to any one of the previous numbered paragraphs 5 to 7 and 10, wherein in said plurality of cycles with high concentration of osmotic agent alternating with said plurality of cycles with low or no concentration of osmotic agent, the low or no concentration of osmotic agent cycle is immediately followed by the high concentration of osmotic agent cycle.

Numbered Paragraph 12. Apparatus according to any one of the previous numbered paragraphs, wherein the control system (74) is configured to leave the treatment fluid in the peritoneal cavity for a dwell time, the dwell time of a treatment fluid with high osmotic agent concentration being different from, and in particular lower than, a dwell time of a treatment fluid with low or no osmotic agent concentration.

Numbered Paragraph 13. Apparatus according to any one of the previous numbered paragraphs, wherein the cycler (3) includes at least two sources (S) of treatment fluid, each source (S) being configured for feeding a treatment fluid different from another treatment fluid from another source (S), the treatment fluids differing at least by the concentration of the osmotic agent, in particular each source (S) being a respective treatment fluid bag.

Numbered Paragraph 14. Apparatus according to any one of the previous numbered paragraphs, wherein the cycler (3) includes a disposable unit (3b) including said patient line (54) configured to be connected to the patient (P) and a peritoneal dialysis unit (3a) including said pump (5) and a compartment configured to receive the disposable unit (3b), the pump (5) being arranged so that when the disposable unit (3b) is disposed within the compartment, the pump (5) cooperates with the disposable unit (3b) to deliver the treatment fluid to and drain the treatment fluid from the peritoneal cavity of the patient via the patient line (54) of the disposable unit (3b).

Numbered Paragraph 15. Apparatus according to the previous numbered paragraph, wherein the disposable unit (3b) includes at least one inlet port (7; 8; 9) connected to said at least a source (S) of treatment fluid and at least one container (41) configured for receiving the treatment fluid from the source (S), in particular the cycler (3) being configured to heat the treatment fluid in the container (41) for receiving the treatment fluid before delivering to the patient via the patient line (54).

Numbered Paragraph 16. Apparatus according to the previous numbered paragraphs 14 or 15, wherein the disposable unit (3b) includes at least two inlet ports (7, 8, 9), said inlet ports being respectively connected to sources (S) of treatment fluid having different concentration of osmotic agent, in particular a different concentration of glucose.

Numbered Paragraph 17. Apparatus according to the previous numbered paragraphs 15 and 16, wherein the cycler (3) includes a valve device (71) to selectively put into communication one of the sources (S) of treatment fluid with the container (41) for receiving the treatment fluid, the control system (74) receiving a target concentration of osmotic agent in the treatment fluid and driving the valve device (71) and the pump (5) to withdraw a prefixed amount of treatment fluid from one of the sources (S) and a prefixed amount of treatment fluid from at least another of the sources (S) to obtain a treatment fluid in the container (41) for receiving the treatment fluid with a concentration of osmotic agent substantially matching the target concentration.

Numbered Paragraph 18. Apparatus according to any one of the previous numbered paragraphs, comprising a sensor (15) for sensing a parameter of the treatment fluid to be delivered to the patient via the patient line (54), the control system (74) being further configured to receive a signal from said sensor (15) and for determining the concentration of osmotic agent in the treatment fluid to be delivered to the patient.

Numbered Paragraph 19. Apparatus according to the previous numbered paragraph 14, comprising at least one source of a first concentrate (50), wherein the disposable unit (3b) includes:

- a water port (43); and
- at least a first inlet port (59) in fluid connection with said source of a first concentrate (50);
- at least a container (41) configured for receiving a treatment fluid prepared by mixing at least water and a first concentrate.

Numbered Paragraph 20. Apparatus according to the previous numbered paragraph, including:

- a water purifier (10) for preparing purified water and feeding to said water port (43),
- a water line (42) for feeding purified water to the water port (43) of the disposable unit (3b), said water line (42) being fluidly connected to the water purifier (10) to receive purified water; and
- a drain line (44) connected to a drain port (19) of the disposable unit (3b) to drain fluids at least from the disposable unit (3b);
- a sensor (15) for detecting a property of a fluid flowing in the drain line (44); and
- the control system (74) being configured to:

  ✓ sending the treatment fluid to be checked contained in the container (41) configured for receiving a treatment fluid into the drain line (44);
  ✓ pushing the treatment fluid to be checked to the sensor (15);

wherein the control system (74) is further configured to achieve the pushing step by sending purified water along the water line (42) and directing said purified water from the water line into the drain line (44), said purified water in the drain line pushing the treatment fluid to be checked so as to reach the sensor (15).

Numbered Paragraph 21. Apparatus according to any one of the previous numbered paragraphs, wherein the control system (74) includes a memory storing a prediction algorithm, the control system (74), based on said prediction algorithm, being configured to determine at least the first concentration of osmotic agent in the first treatment fluid, the second concentration of osmotic agent in the second treatment fluid, and the third concentration of osmotic agent in the third treatment fluid.

Numbered Paragraph 22. Apparatus according to the previous numbered paragraph, wherein the control system (74), based on said prediction algorithm, is configured to determine at least a first dwell time for the first treatment fluid, a second dwell time for the second treatment fluid, and a third dwell time for the third treatment fluid.

Numbered Paragraph 23. Apparatus according to the previous numbered paragraphs 21 or 22, wherein the control system (74) receives as input a desired total UF volume to be removed at the end of the treatment session and/or a desired total small solute removal, e.g. urea, to be removed at the end of said treatment session and provides said first, second and third concentrations for the osmotic agent as an output.

Numbered Paragraph 24. Apparatus according to any of the previous numbered paragraphs 21 to 23, wherein the control system (74) receives as input the total number N of cycles of the treatment session and provides as an output, based on said prediction algorithm, a corresponding concentration of osmotic agent and a corresponding dwell time for each of the N cycles to achieve a desired total UF volume and total small solute removal targets at the end of the treatment session.

Numbered Paragraph 25. Apparatus according to any of the previous numbered paragraphs 21 to 24, wherein the prediction algorithm includes a modified three pore model based on the equation:

$$\frac{\mathrm{d}V_d}{\mathrm{d}t} = J_{v,C} + J_{v,S} + J_{v,L} - L + J_{\text{fill}} - J_{\text{drain}}$$

wherein:

$J_{v,C}$ is the a net flow of water (in mL/min) across aquaporins;
$J_{v,S}$ is the a net flow of water (in mL/min) across highly selective pathways or "small pores";
$J_{v,L}$ is the a net flow of water (in mL/min) across weakly selective pathways or "large pores";
$L$ is the a net lymphatic clearance (in mL/min) from the peritoneal cavity to the circulation;
$J_{\text{fill}}$ is the a flow of volume (in mL/min) to the source of treatment fluid; and
$J_{\text{drain}}$ is the a flow of volume (in mL/min) from the source of treatment fluid.

**Claims**

1. An apparatus for peritoneal dialysis (1) comprising:

   an automated peritoneal dialysis cycler (3) including:

      a patient line (54) configured to be placed in communication with a peritoneal cavity of a patient,
      a water port (43) for receiving water,
      an inlet port (59) in fluid communication with a source of concentrate (50) that includes an osmotic agent,
      a container (41) fluidly coupled to the water port, the inlet port, and the patient line, and
      at least one pump (69, 70) for preparing treatment fluid and delivering the treatment fluid to the patient through the patient line; and

   a control system (74) configured to operate the cycler (3) to deliver the treatment fluid, wherein the control system is programmed to:

      create, at a first time, a first treatment fluid having a first concentration of the osmotic agent by:

         causing the at least one pump to pump a first amount of water from the water port to the container,
         causing the at least one pump to pump a first amount of the concentrate from the inlet port to the container, and
         causing the first amount of water to mix with the first amount of concentrate to create the first treatment fluid, and

create, at a second time that occurs after the first treatment fluid has been provided to the patient, a second treatment fluid having a second concentration of the osmotic agent by:

causing the at least one pump to pump the first amount or a second amount of water from the water port to the container,
causing the at least one pump to pump a second amount of the concentrate from the inlet port to the container, and
causing the first amount or second amount of water to mix with the second amount of concentrate to create the second treatment fluid,

wherein the second concentration of the osmotic agent in the second treatment fluid is greater than the first concentration of the osmotic agent in the first treatment fluid.

2. The apparatus according to claim 1, further comprising:

a water purifier (10) for preparing purified water; and
a water line (42) fluidly coupling the water purifier to the water port of the automated peritoneal dialysis cycler.

3. The apparatus according to claims 1 or 2, wherein the automated peritoneal dialysis cycler (3) further includes:

a drain port (19) fluidly coupled to the at least one pump;
a drain line (44) fluidly coupled to the drain port (19); and
a sensor (15) located along the drain line, the sensor configured to detect a property of the treatment fluid.

4. The apparatus according to claim 3, wherein the control system is further configured to:

cause the at least one pump to send a portion of the mixed treatment fluid to the drain line; and
push the portion of the mixed treatment fluid to the sensor,
wherein the control system is configured to achieve the pushing step by causing the at least one pump to push purified water from the water line to the drain line, the purified water in the drain line pushing the mixed treatment fluid to the sensor.

5. The apparatus according to claim 4, wherein the control system is further configured to:

use the property measured by the sensor to determine whether an osmatic concentration within the mixed first or second treatment fluid matches a respective programmed osmatic concentration;
when the osmatic concentration within the mixed first or second treatment fluid matches the respective programmed osmatic concentration, cause the at least one pump to deliver at least a portion of the mixed treatment to the patient via the patient line; and
when the osmatic concentration within the mixed first or second treatment fluid does not match the respective programmed osmatic concentration, cause the at least one pump to pump additional water or concentrate to the container to cause the osmatic concentration within the mixed first or second treatment fluid to more closely match the respective programmed osmatic concentration.

6. The apparatus according to claim 3, wherein the sensor includes at least one of a glucose concentration sensor or a conductivity sensor.

7. The apparatus according to claims 1, 2, or 3, wherein at least one of the water line, the patient line, the container, the drain line, and a pump chamber of the at least one pump is part of a disposable unit (3b) that is operable with the automated peritoneal dialysis cycler (3).

8. The apparatus according to claim 1, wherein the control system is configured to:

cause the first amount of water to mix with the first amount of concentrate to create the first treatment fluid by repeatedly withdrawing and sending back to the container some of the water mixed with the first amount of concentrate; and
cause the first amount or second amount of water to mix with the second amount of concentrate to create the second treatment fluid by repeatedly withdrawing and sending back to the container some of the water mixed with the second amount of concentrate.

9. The apparatus according to claim 1, wherein the control system is configured to further cause the first amount of water to mix with the first amount of concentrate by causing the at least one pump to pump additional water from the water port to the container after causing the at least one pump to pump the first amount of the concentrate; and wherein the control system is configured to further cause the first or second amount of water to mix with the second amount of concentrate by causing the at least one pump to pump additional water from the water port to the container after causing the at least one pump to pump the second amount of the concentrate.

10. The apparatus according to claim 1, wherein the osmotic agent is, or includes, glucose, L- carnitine, glycerol, or combinations thereof, in particular the osmotic agent being or including glucose.

11. The apparatus according to claim 1, wherein the control system (74) is programmed to run one or more cycles of a first peritoneal dialysis treatment using the first treatment fluid followed by one or more additional cycles of the first peritoneal dialysis treatment or a second peritoneal dialysis treatment using the second treatment fluid.

12. The apparatus according to claim 1, wherein the first treatment fluid includes the osmotic agent having a concentration of 2% by weight or lower, optionally 1.5% by weight or lower, e.g., 0.5% by weight or lower, the osmotic agent being in particular glucose.

13. The apparatus according to claims 1 or 12, wherein the second treatment fluid includes the osmotic agent having a concentration of 3% by weight or higher, optionally 4% by weight or higher, e.g., 5% by weight or higher, the osmotic agent being in particular glucose.

14. The apparatus according to claim 1, wherein the control system (74) is configured to leave the treatment fluid in the peritoneal cavity for a dwell time, the dwell time of the second treatment fluid with the higher osmotic agent concentration being different from, and in particular lower than, a dwell time of the first treatment fluid with the lower osmotic agent concentration.

15. The apparatus according to claim 1, wherein the automated peritoneal dialysis cycler (3) further includes a second inlet port (60) in fluid communication with a source of second concentrate (53) that includes a different concentration of the osmotic agent or a second osmotic agent, and wherein the control system (74) is configured to create the second treatment fluid by:

causing the at least one pump to pump the first amount or the second amount of water from the water port to the container,
causing the at least one pump to pump the second amount of the second concentrate from the second inlet port to the container, and
causing the first amount or second amount of water to mix with the second amount of concentrate to create the second treatment fluid.

FIG.1

EP 4 445 924 A2

FIG.1A

FIG.1B

FIG.2

EP 4 445 924 A2

# FIG.3

EP 4 445 924 A2

LARGE

SMALL

TRANSCELLULAR

FIG.4

FIG.5

FIG.6

FIG.7

FIG.8

FIG.9

FIG.10

FIG.11

FIG.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0402505 A
- US 5643201 A
- WO 2012129501 A
- US 2008125693 A
- US 2014221910 A
- US 20107010426 A
- US 2010010426 A

### Non-patent literature cited in the description

- **KESHAVIAH P ; EMERSON PF ; VONESH EF ; BRANDES JC.** Relationship between body size, fill volume, and mass transfer area coefficient in peritoneal dialysis. *J Am Soc Nephrol,* 1994, vol. 4, 1820-1826
- **TWARDOWSKI ZJ ; PROWANT BF ; NOLPH KD ; MARTINEZ AJ ; LAMPTON LM.** High volume, low frequency continuous ambulatory peritoneal dialysis. *Kidney Int,* 1983, vol. 23, 64-70